# EUROPÄISCHE PATENTSCHRIFT

(11) **EP 2 868 314 B1**
(45) Veröffentlichungstag und Bekanntmachung des Hinweises auf die Patenterteilung: **30.05.2018**
(21) Anmeldenummer: 13192533.1
(22) Anmeldetag: 12.11.2013
(51) Int. Cl.: A61K 9/00, A61K 47/38, A61J 1/00, A61K 36/752, A61K 36/899, A61K 36/00, A61K 36/53, A61K 36/534, A61K 36/54, A61L 27/12, A61L 33/00, A61M 15/08, A61K 36/185, A61K 36/61, A61M 15/00, A23L 5/00, A23L 27/12, A23L 33/00

(54) **Zusammensetzung zur Verwendung in einer Inhalationsvorrichtung und hierfür einsetzbare Inhalationsvorrichtung**
Composition for use in an inhalation device and inhalation device that can be used for same
Composition destinée à être utilisée dans un dispositif d'inhalation et dispositif d'inhalation pouvant être utilisé à cet effet

(30) Priorität: 31.10.2013 EP 13005165
(43) Veröffentlichungstag der Anmeldung: 06.05.2015
(73) Patentinhaber: AromaStick AG, 9493 Mauren (LI)
(72) Erfinder: Singer, Jürg, 9493 Mauren (LI)
(74) Vertreter: Von Rohr Patentanwälte Partnerschaft mbB

(56) Entgegenhaltungen:
- EP-A2- 0 848 959
- GB-A- 576 340
- GB-A- 191 306 078
- US-A1- 2006 191 546
- US-A1- 2008 187 609

## Beschreibung

Die vorliegende Erfindung betrifft das Gebiet der prophylaktischen oder therapeutischen Behandlung von Erkrankungen mittels inhalativer Applikation von ätherischen Ölen bzw. Ölgemischen.

Die vorliegende Erfindung betrifft einen speziell ausgebildeten Inhalationsstift (synonym auch als Inhalier- bzw. Inhalationsvorrichtung, Inhalierstift, Inhaler, Riechstift, Duftstift oder dergleichen bezeichnet) nach dem Oberbegriff des diesbezüglich unabhängigen Patentanspruchs sowie ein Verfahren zu dessen Montage. Insbesondere betrifft die vorliegende Erfindung gemäß diesem Erfindungsaspekt einen Inhalationsstift mit einer langgestreckten Gehäusehülse und einem stabförmigen Speichermittel zum Speichern mindestens eines ätherischen Ol(gemisch)s, wobei die Gehäusehülse einen inneren Aufnahmeraum zur Aufnahme des Speichermittels aufweist.

Gleichermaßen betrifft die vorliegende Erfindung ein Verfahren zur Montage, insbesondere zum Befüllen, eines solchen Inhalationsstifts.

Grundsätzlich ist die Anwendung von Aromatherapien auf Basis von ätherischen Ölen seit Beginn des 20. Jahrhunderts bekannt und wird vornehmlich zu Zwecken der Entspannung bzw. als Entspannungstechnik, zunehmend aber auch zur Behandlung von Erkrankungen eingesetzt. Insbesondere wird in der Aromatherapie eine Alternative zu klassischen schulmedizinischen Ansätzen gesehen; sie wird teilweise aber auch in Ergänzung zur schulmedizinischen Behandlung eingesetzt.

Die Aromatherapie basiert vorwiegend auf der Anwendung bzw. Applikation ätherischer Öle, welche zahlreiche volatile bzw. flüchtige Duftstoffe auf Basis von sekundären Pflanzenstoffen mit unterschiedlicher Wirkung auf den Köper enthalten. Insgesamt existiert eine Vielzahl verschiedener ätherischer Öle aus verschiedenen Pflanzen. Dementsprechend können mit Hilfe verschiedener ätherischer Öle bzw. der darin enthaltenen Duftstoffe unterschiedliche Wirkungen erzielt werden. Ätherische Öle können sowohl eine Wirkung in Bezug auf die Physiologie als auch in Bezug auf die Psychologie von Personen bzw. Patienten entfalten.

Nachteilig ist jedoch, dass ätherische Öle im Allgemeinen als Einzelöle angeboten werden, nicht jedoch als Kombinations- oder Komplexpräparate. Die große Vielzahl an angebotenen ätherischen Ölen mit unterschiedlichen Wirkungen ist für den Patienten bzw. Verbraucher insgesamt unüberschaubar und erfordert für eine wirkungsvolle Therapie die Hinzuziehung eines Fachmanns bzw. Aromatherapeuten. Insgesamt existieren kaum Konzepte zur Bereitstellung von Aromaölpräparaten bzw. Zusammensetzungen, welche bereits anwendungsfertig vorliegen und in zielgerichteter Weise zur Behandlung einzelner Erkrankungen eingesetzt werden können.

Die Durchführung von Aromatherapien bzw. die Anwendung der ätherischen Öle ist äußerst vielfältig. Eine Möglichkeit besteht beispielsweise darin, die Aromaöle bzw. ätherischen Öle direkt auf die Haut aufzubringen, was teilweise auch mit (Aromaöl-) Massagen verbunden wird. Der direkte Auftrag von ätherischen Ölen auf die Haut führt jedoch nicht immer zur erwünschten Wirkung. Insbesondere kann es zu Nebenwirkungen, wie Hautreizungen oder allergischen Reaktionen, kommen. Darüber hinaus ist die Wirkeffizienz bei einem Auftragen auf die Haut nicht immer optimal bzw. zu gering.

Oftmals ist es auch vorgesehen, eine Aromatherapie nach Art eines Dampfbades zu applizieren, d. h. eine Schüssel mit heißem Wasser zusammen mit ätherischen Ölen zu befüllen und die aufsteigenden Dämpfe, vorzugsweise mit einem Handtuch über dem Kopf, zu inhalieren. Diese Methode ist einerseits in der Handhabung verhältnismäßig unkomfortabel, andererseits können die Dämpfe in die Augen gelangen, so dass die Schleimhäute der Augen mitunter gereizt werden. Zudem besteht durch das heiße Wasser die Gefahr vor Verbrühungen.

Weiterhin kann eine Aromatherapie auch über die Raumluft, beispielweise durch das Aufstellen von Duftlampen, welche mit dem jeweiligen Aromaöl bzw. ätherischen Öl beschickt werden, erfolgen. Auch diese Methode zeichnet sich oftmals durch eine zu geringe Wirkeffizienz aus. Darüber hinaus ist es nicht immer erwünscht, dass der ganze Raum bzw. die Raumluft mit den Duftstoffen der ätherischen Öle angereichert wird. Auch ist problematisch bzw. wenig praktikabel, dass eine solche Aromatherapie nicht ortsunabhängig erfolgen kann.

Zudem kann die Verabreichung der einer Aromatherapie zugrundeliegenden Komponenten bzw. die Aromatherapie als solche über inhalative Maßnahmen bzw. Inhalationsvorrichtungen erfolgen. Im Allgemeinen werden dazu apparativ aufwendige Inhaliergeräte eingesetzt. Auch ist es möglich, ätherische Öle mit Hilfe von sogenannten Inhalationsstiften bzw. Inhalierstiften inhalativ zu applizieren. Die aus dem Stand der Technik bekannten Inhalationsstifte sind diesbezüglich jedoch oftmals nachteilig ausgestaltet bzw. ungeeignet, wie nachfolgend im Detail geschildert.

Ein Inhalationsstift weist konstruktiv im Allgemeinen ein Speichermittel aus einem Flüssigkeit aufnehmenden, komprimierbaren Material auf, bei dem es sich beispielsweise um ein Vlies oder dergleichen handeln kann, das mit einer einen bestimmten Wirkstoff abgebenden Flüssigkeit getränkt ist. Zur Benutzung wird das Vorderteil des Inhalationsstiftes, an dem sich die Inhalieröffnung befindet, so weit in die Nase des Nutzers eingeführt bzw. an diese herangeführt, dass die Wirkstoffe möglichst vollständig über die Nase aufgenommen werden können.

Bei den aus der US 2008/0187609 A bekannten Inhalationsstiften ist das Speichermittel in die Gehäusehülse des Inhalationsstiftes frei beweglich eingebracht. Hierdurch kommt das getränkte Speichermittel mit dem Material der Gehäusehülse über eine große Fläche permanent in Kontakt. Durch den direkten Kontakt mit der Hülse diffundiert ein nicht unerheblicher Teil des Inhalationsmittels aus dem getränkten Speichermittel in das Material des Primärbehälters, der in der Regel aus Kunststoff besteht. Diese Problematik ergibt sich insbesondere bei Lösungen mit ätherischen Ölen. Derartige Inhalationsstifte sind deshalb mitunter nicht für eine Aromatherapie auf Basis von ätherischen Ölen geeignet.

Weiterhin gestaltet sich das Befüllen des sich frei bewegenden Speichermittels des bekannten Inhalationsstiftes problematisch. Üblicherweise wird das Speichermittel vor dem Einsetzen getränkt. Allerdings können durch das anschließende Hantieren des getränkten Speichermittels bis zur Einführung in die Gehäusehülse hygienische Probleme und Sterilitätsprobleme entstehen. Nicht selten geht Inhaliermittel, mit dem das Speichermittel getränkt worden ist, beim Transportieren des Speichermittels zur Gehäusehülse verloren. Dabei kann es auch zu einer Verschmutzung auf der Außenseite der Gehäusehülse kommen. Auch ist eine präzise Befüllung mit einem definierten Volumen bzw. einer definierten Menge der ätherischen Öle bei einem Tränken in der Aromalösung nicht möglich.

Zudem weisen Inhalationsstifte des Standes der Technik nicht immer optimale Abgabe- bzw. Applikationseigenschaften auf. So ist das mitunter großflächige Anliegen des Speichermittels an der Gehäusehülse einer optimalen Freisetzung volatiler Komponenten abträglich.

Insgesamt kann somit mit den aus dem Stand der Technik bekannten Methoden zur Aromatherapie nicht immer ein Wirkoptimum erzielt werden, sowohl was geeignete Zusammensetzungen als solche als auch die entsprechenden Applikationsvorrichtungen, insbesondere geeignete Inhaltsstifte, anbelangt.

Somit liegt der vorliegenden Erfindung die Aufgabe zugrunde, aromatherapeutisch wirksame Zusammensetzungen in einem Inhalationsstift zur zielgerichteten Behandlung von verschiedenen Erkrankungen bereitzustellen, welche die zuvor genannten Nachteile des Standes der Technik wenigstens teilweise vermeiden oder aber zumindest teilweise abschwächen.

Insbesondere liegt der vorliegenden Erfindung diesbezüglich die Aufgabe zugrunde, Zusammensetzungen auf Basis von ätherischen Ölen in einem Inhalationsstift bereitzustellen, welche zur effektiven und zielgerichteten Behandlung von Erkrankungen geeignet sind. Der vorliegenden Erfindung liegt die Aufgabe zugrunde, einen Inhalationsstift bereitzustellen, welcher die zuvor genannten Nachteile des Standes der Technik wenigstens teilweise vermeidet oder aber zumindest teilweise abschwächt. Insbesondere liegt der vorliegenden Erfindung diesbezüglich die Aufgabe zugrunde, einen Inhalationsstift bereitzustellen, welcher sich durch eine vereinfachte Handhabung im Rahmen des Herstellungsprozesses auszeichnet und insbesondere eine vereinfachte bzw. verbesserte Befüllung mit einer Zusammensetzung auf Basis von ätherischen Ölen ermöglicht. Darüber hinaus sollen Sterilitätsprobleme vermieden bzw. vermindert werden.

Zur Lösung der zuvor geschilderten Aufgabe schlägt die vorliegende Erfindung gemäß einem ersten Erfindungsaspekt einen speziell ausgebildeten Inhalationsstift nach Anspruch 1 vor; weitere vorteilhafte Ausgestaltungen sind Gegenstand der diesbezüglichen abhängigen Ansprüche.

Gleichermaßen ist gemäß einem weiteren Erfindungsaspekt Gegenstand der vorliegenden Erfindung ein Verfahren zur Montage eines erfindungsgemäßen Inhalationsstifts nach den diesbezüglichen Ansprüchen.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Aspekt der vorliegenden Erfindung - ein Inhalationsstift 1 zur Inhalation durch die Nase mit einer langgestreckten Gehäusehülse 2 und einem stabförmigen Speichermittel 3 zum Speichern mindestens eines ätherischen Öls, wobei die Gehäusehülse 2 einen inneren Aufnahmeraum 4 zur Aufnahme des Speichermittels 3 aufweist, wobei innenseitig in der Gehäusehülse 2 wenigstens ein in radialer Richtung in den Aufnahmeraum 4 ragender Fixiervorsprung zur reibschlüssigen Fixierung des Speichermittels 4 in der Gehäusehülse 2 vorgesehen ist, und wobei zum Verschluss der Gehäusehülse 2 eine in den hinteren Hülsenbereich 10 einsetzbare Stopfenhülse 17 vorgesehen ist, und dass in der Stopfenhülse 17 mit endseitigen Auflaufanschlägen 21 versehene, als Rippen 20 ausgebildete Fixiervorsprünge zur endseitigen Fixierung des Speichermittels 3 vorgesehen sind.

Im Rahmen der vorliegenden Erfindung ist ein reversibles Speichern, insbesondere eine reversible Aufnahme, insbesondere derart zu verstehen, dass das mit der volatilen Flüssigkeit versehene bzw. beaufschlagte Speichermittel bei Anwendung des Inhalationsstifts die in der volatilen Flüssigkeit enthaltenen insbesondere volatilen Wirk- bzw. Inhaltsstoffe freizusetzen bzw. abzugeben imstande ist, so dass diese ihre aromatherapeutische Wirkung entfalten können.

Die eingangs geschilderte Aufgabe ist bei einem Inhalationsstift der vorgenannten Art erfindungsgemäß dadurch gelöst, dass innenseitig in der Gehäusehülse wenigstens ein insbesondere in radialer Richtung in den Aufnahmeraum ragender Fixiervorsprung zur reibschlüssigen Fixierung des Speichermittels in der Gehäusehülse vorgesehen ist. Durch den Fixiervorsprung, der in radialer Richtung in den Aufnahmeraum hineinragt, ergeben sich verschiedene wesentliche Vorteile. Zunächst einmal wird durch den in radialer Richtung in den Aufnahmeraum ragenden Fixiervorsprung der lichte Durchmesser innerhalb des Aufnahmeraums verringert. Der durch den Fixiervorsprung verringerte lichte Durchmesser des Aufnahmeraums ist kleiner als der Außenmesser des stabförmigen Speichermittels, so dass sich nach Einsetzen des Speichermittels in die Gehäusehülse ein Reibschluss zwischen dem Fixiervorsprung und dem Speichermittel ergibt. Auf diese Weise ergibt sich eine sichere Fixierung und lagestabile Anordnung des Speichermittels in der Gehäusehülse. Durch die lagestabile Anordnung des Speichermittels in der Gehäusehülse ist im Übrigen nicht nur sichergestellt, dass sich das Speichermittel während der Handhabung des Inhalationsstiftes in der Gehäusehülse nicht bewegt, was vom Benutzer als unangenehm empfunden wird. Es ist darüber hinaus möglich, das unbeladene Speichermittel zunächst in die Gehäusehülse einzubringen und erst anschließend zu befüllen, da eine definierte und fixierte Anordnung des Speichermittels in der Gehäusehülse gewährleistet ist und somit nicht befürchtet werden muss, dass die in das Speichermittel einzubringende Flüssigkeit an einer Stelle in die Gehäusehülse eingebracht wird, an der sich kein Speichermittel befindet. Daraus resultiert letztlich auch, dass nicht nur eine sehr exakte Dosierung der Inhalationsflüssigkeit möglich ist, es werden auch Hygiene- und Sterilitätsprobleme vermieden. Schließlich ist aufgrund der erfindungsgemäß vorgesehenen Ausbildung der Innenstruktur der Gehäusehülse gewährleistet, dass das Speichermittel nur bereichsweise an der inneren Wandung des Aufnahmeraums anliegt, so dass die Diffusionsproblematik beim erfindungsgemäßen Inhalationsstift allenfalls geringfügig auftritt.

Im Hinblick auf die Befüllung des Speichermittels mit der vorzugsweise volatilen Flüssigkeit, insbesondere der Zusammensetzung nach der vorliegenden Erfindung, ist die erfindungsgemäße Fixierung des Speichermittels zudem dahingehend vorteilhaft, dass sie es ermöglicht, die Wirkstofflösung ("ätherische Öle") auf Basis der vorzugsweise volatilen Flüssigkeit direkt in das Speichermittel einzubringen. Im Stand der Technik hingegen findet oftmals ein nicht zielgerichtetes Aufbringen bzw. ein "Aufgießen" von oben auf das Speichermittel statt, was dazu führt, dass ein Teil der Tränklösung nicht schnell genug vom Speichermittel aufgesaugt wird oder auf sonstige Art und Weise unsachgemäß in der Hülse verteilt wird.

Während es bereits grundsätzlich ausreicht, zur Fixierung des Speichermittels und zu dessen zumindest bereichsweisen Beabstandung zur Innenwandung des Aufnahmeraums nur einen Fixiervorsprung vorzusehen, hat es sich als besonders vorteilhaft erwiesen, in der Gehäusehülse eine Mehrzahl von Fixiervorsprüngen vorzusehen. Diese sind bevorzugt derart ausgebildet, dass das Speichermittel, dessen Durchmesser an die Abmaße der Fixiervorsprünge anzupassen ist, ausschließlich an den Fixiervorsprüngen anliegt, ohne an dem übrigen Innenwandungsbereich des Aufnahmeraums anzuliegen. Insofern ergibt sich eine überwiegende Berührungsfreiheit, so dass Diffusion an diesen Stellen nicht auftreten kann.

Bei einer besonders vorteilhaften Ausgestaltung der Erfindung ist das Speichermittel durch insbesondere über den Umfang des Aufnahmeraums gleich beabstandete Fixiervorsprünge zentriert im Aufnahmeraum angeordnet. Durch die zentrierte Anordnung über die Fixiervorsprünge ergibt sich eine lagedefinierte, mittige Anordnung des Speichermittels in der Gehäusehülse, so dass es möglich ist, die Inhalierflüssigkeit über eine mittige Nasenöffnung der Gehäusehülse in das Speichermittel einzubringen.

Als besonders zweckmäßig hat es sich in diesem Zusammenhang erwiesen, dass die Fixiervorsprünge als langgestreckte Rippen ausgebildet sind. Durch die rippenartige Ausbildung der Fixiervorsprünge ergibt sich ein linienförmiger Kontakt zwischen dem Speichermittel und den Fixiervorsprüngen, was die reibschlüssige Verbindung und damit die lagesichere Anordnung des Speichermittels in der Gehäusehülse verbessert. Dies ist insbesondere beim Befüllvorgang von Bedeutung. Allerdings darf darauf hingewiesen werden, dass es grundsätzlich auch möglich ist, statt einzelner langgestreckter Rippen eine Mehrzahl von in Hülsenlängsrichtung hintereinander angeordnete Einzelvorsprüngen vorzusehen.

Letztlich hat es sich aber als besonders bevorzugt erwiesen, drei langgestreckte Rippen in der Gehäusehülse, die jeweils über 120° voneinander beabstandet sind, über den Umfang des Aufnahmeraums verteilt an dessen Innenwandung vorzusehen, um einerseits eine gute Fixierung und Zentrierung des Speichermittels zu erzielen und um andererseits eine nur geringe Diffusion in die Gehäusewandung zu ermöglichen.

Die Gehäusehülse selbst, die vorzugsweise aus einem Kunststoff besteht, weist einen vorderen Hülsenbereich und einen hinteren Hülsenbereich auf. Der vordere, langgestreckte Hülsenbereich dient als Inhalationsbereich, während der hintere Hülsenbereich als Griffbereich und damit zu Halten des Inhalationsstiftes bei der Handhabung dient. Eine derartige Gehäusehülse lässt sich relativ einfach und kostengünstig herstellen, wobei aus handhabungstechnischen Gründen der hintere Hülsenbereich einen etwas größeren Außendurchmesser als der vordere Hülsenbereich haben sollte.

In konstruktiver Ausgestaltung der Gehäusehülse ist am vorderen Ende des vorderen Hülsenbereichs wenigstens eine, vorzugsweise eine einzige in den Aufnahmeraum führende, mittige Nasenöffnung vorgesehen. Darüberhinaus bietet es sich an, wenn gleichfalls am vorderen Hülsenbereich, jedoch vom vorderen Ende hinreichend beabstandet, wenigstens eine insbesondere radial in den Aufnahmeraum führende Seitenöffnung vorgesehen ist. Diese Ausgestaltung hat sich aus handhabungstechnischer Sicht aus zwei Gründen als vorteilhaft erwiesen. Bei der Handhabung des Inhalationsstiftes wird das vordere Ende des vorderen Hülsenbereichs in die Nase des Nutzers eingeführt bzw. im Bereich der Nase positioniert (insbesondere im Bereich der Nasenlöcher). Über die Nasenöffnung des Inhalationsstifts können dann die Wirkstoffe aufgenommen werden. Die Beabstandung der Seitenöffnung von der vorderen Nasenöffnung sollte dabei so sein, dass sich im eingeführten Zustand die Seitenöffnung außerhalb der Nase des Nutzers befindet. Hierdurch wird dann gewährleistet, dass hinreichend Luft bei der Nutzung des Inhalationsstiftes über die Seitenöffnung angesogen werden kann. Im Übrigen ist durch die mittige Anordnung der Nasenöffnung und die gleichzeitige mittige Anordnung des Speichermittels in der Gehäusehülse gewährleistet, dass eine definierte Zuführung der Inhalierflüssigkeit bzw. der Zusammensetzung nach der Erfindung zum Speichermittel über die Nasenöffnung möglich ist. Durch die Beabstandung des Speichermittels zur Wandung des Inhalationsstifts ist zudem die Freisetzung der Zusammensetzungen verbessert, was insbesondere einhergehend mit strömungstechnischen Vorteilen (d. h. geringem Strömungswiderstand bzw. geringem Druckverlust) zu einer insgesamt verbesserten Verabreichung der nachfolgend beschriebenen Zusammensetzungen führt.

Um insbesondere für das Beladen des Speichermittels eine lagestabile Anordnung des Speichermittels in der Gehäusehülse zu gewährleisten, ist bei einer bevorzugten Ausgestaltung der Erfindung vorgesehen, dass die Rippen in der Gehäusehülse im Bereich des vorderen Endes des vorderen Hülsenbereichs vorgesehen sind. Dabei können sich die Rippen letztlich von der vorderen Stirnwandung der Gehäusehülse in den Aufnahmeraum in axialer Richtung erstrecken.

Um außerdem eine lagedefinierte Anordnung des Speichermittels in der Gehäusehülse zu gewährleisten, weisen die Rippen jeweils einen Anschlag zur endseitigen Anlage des Speichermittels auf. Durch den Anschlag im Bereich der Rippen ist gewährleistet, dass das Speichermittel nur bis zu einem bestimmten Punkt, nämlich dem Anschlagspunkt, in die Gehäusehülse eingesetzt werden kann.

In diesem Zusammenhang kann im Übrigen vorgesehen sein, dass die Rippen jeweils mit einer endseitigen Auflaufschräge versehen sind, um das problemlose Einsetzen des stabförmigen Speichermittels in die Gehäusehülse und das Aufschieben des Speichermittels auf die Rippen zur reibschlüssigen Halterung bis zum Erreichen des Anschlags zu gewährleisten.

Das Gehäuse des erfindungsgemäßen Inhalationsstiftes ist bevorzugt zumindest zweiteilig aufgebaut und weist neben der Gehäusehülse einen Stopfen oder Verschluss auf, der bevorzugt als in den hinteren Hülsenbereich einsetzbare bzw. eingesetzte Stopfenhülse ausgebildet ist. Zum Verschluss der Gehäusehülse würde an sich auch eine Kappe oder ein sonstiger Deckel ausreichen. Durch den zuvor geschilderten Einsatz eines Stopfens kann der Inhalationsstift nach der vorliegenden Erfindung durch den Anwender bzw. Benutzer nicht ohne Weiteres geöffnet werden, so dass das Speichermittel nicht entfernt, unsachgemäß verwendet oder befüllt sowie verschmutzt werden kann. Erfindungsgemäß ist es vorgesehen, eine Stopfenhülse zu verwenden, an der ebenfalls Fixiervorsprünge zur endseitigen Fixierung des Speichermittels vorgesehen sind. Die Fixiervorsprünge sind erfindungsgemäß mit endseitigen Auflaufanschlägen versehen. Diese Ausgestaltung erleichtert den Zusammenbau des erfindungsgemäßen Inhalationsstiftes erheblich. So ist es möglich, das Speichermittel zunächst in die Gehäusehülse einzuschieben, ohne dass es dann bereits zu einem Kontakt des einen Endes des Speichermittels mit den Anschlägen an den innenseitig in der Gehäusehülse vorgesehenen Rippen kommen muss. Durch Aufsetzen der Stopfenhülse wird über die Auflaufanschläge das Speichermittel in seine endgültige Montageposition gedrückt, so dass sich anschließend an beiden Enden des Speichermittels eine lagesichere Halterung und Fixierung zum einen über die Fixiermittel der Gehäusehülse und zum anderen über die Fixiermittel an der Stopfenhülse ergibt.

Um eine einfache Verbindung zwischen der Stopfenhülse und der Gehäusehülse zu erreichen, ist es vorgesehen, dass die beiden Bauteile miteinander verrastet sind. Bei einer bevorzugten Ausgestaltung sind außenseitig an der Stopfenhülse ein umlaufender Rastvorsprung und innenseitig am hinteren Hülsenbereich eine umlaufende Nut oder Stufe vorgesehen, die von dem Rastvorsprung hintergriffen wird.

Um einen ungewollten Wirkstoffaustritt aus dem Inhalationsstift bei dessen Nicht-Nutzung zu vermeiden, ist eine Verschlusskappe zum Aufsetzen auf den vorderen Hülsenbereich vorgesehen. Die Verschlusskappe ist dabei derart ausgebildet, dass im aufgesetzten Zustand die Nasen- und Seitenöffnungen verschlossen sind. Bei einer einfachen Ausgestaltung ist am Übergang vom vorderen Hülsenbereich zum hinteren Hülsenbereich ein Außengewinde zum Verschrauben der Verschlusskappe mit der Gehäusehülse vorgesehen. Zu dem Außengewinde an der Gehäusehülse korrespondiert ein Innengewinde der Verschlusskappe, wobei die Schraubverbindung insgesamt so ausgebildet ist, dass ein zumindest im Wesentlichen luftdichter Verschluss und eine feste Halterung der Verschlusskappe an der Gehäusehülse bereits nach einer Drehung der Verschlusskappe gegenüber der Gehäusehülse von weniger als 360°, insbesondere zwischen 180° und 330°, erfolgt.

Es wird ausdrücklich darauf hingewiesen, dass alle vorgenannten Intervalle auch alle Zwischenintervalle und im Übrigen alle Einzelwerte, die innerhalb der Intervallgrenzen liegen, beinhalten und als erfindungswesentlich angesehen werden, auch wenn diese Einzelwerte oder Zwischenintervalle im Einzelnen nicht angegeben sind.

Wie nachfolgend ausgeführt, ist es erfindungsgemäß insbesondere vorgesehen, dass der Inhalationsstift eine Zusammensetzung, wie sie nachfolgend beschrieben wird, enthält. Erfindungsgemäß ist das Speichermittel mit einer volatilen Flüssigkeit, insbesondere der Zusammensetzung, beaufschlagt bzw. versehen.

Gleichermaßen kann es erfindungsgemäß vorgesehen sein, dass das Speichermittel des erfindungsgemäßen Inhalationsstifts mit einer vorzugsweise volatilen Flüssigkeit, insbesondere einer Zusammensetzung nach der Erfindung, beaufschlagt und/oder versehen ist.

Weitere Merkmale, Vorteile und Anwendungsmöglichkeiten der vorliegenden Erfindung ergeben sich aus der nachfolgenden Beschreibung von bevorzugten Ausführungsformen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder ihrer Rückbeziehung.

Es zeigt:
- Fig. 1: eine perspektivische Ansicht eines erfindungsgemäßen Inhalationsstiftes,
- Fig. 2: eine Schnittansicht des Inhalationsstiftes aus Fig. 1,
- Fig. 3: eine perspektivische Schnittdarstellung einer Gehäusehülse eines erfindungsgemäßen Inhalationsstiftes,
- Fig. 4: eine perspektivische Ansicht einer Stopfenhülse des erfindungsgemäßen Inhalationsstiftes,
- Fig. 5: eine weitere perspektivische Ansicht der Stopfenhülse aus Fig. 4 und
- Fig. 6: eine perspektivische Schnittdarstellung des erfindungsgemäßen Inhalationsstiftes im geschlossenen Zustand ohne Speichermittel.

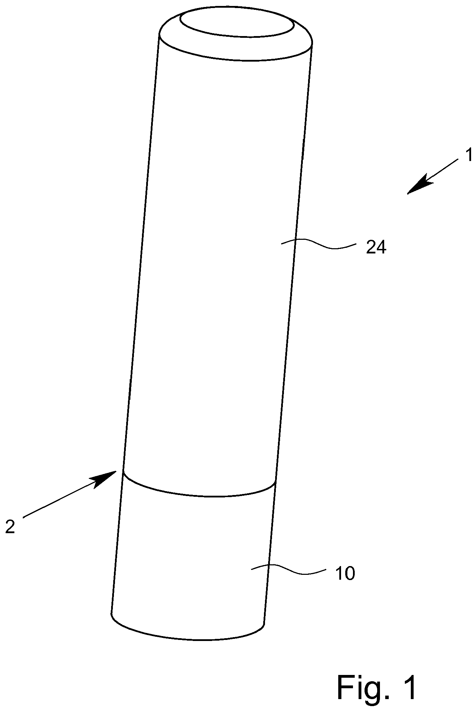

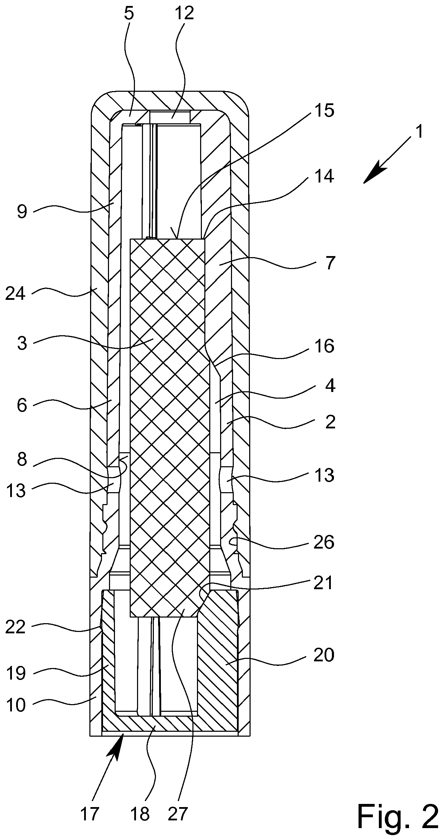

In den Fig. 1 und 2 ist ein Inhalationsstift 1 dargestellt. Der Inhalationsstift 1 weist eine langgestreckte Gehäusehülse 2 und stabförmiges Speichermittel 3 aus einem komprimierbaren Material auf. Die Gehäusehülse 2 besteht vorliegend aus einem Kunststoffmaterial. Das Speichermittel 3 dient zum Speichern einer mindestens einen Wirkstoff aufweisenden, vorzugsweise volatilen Flüssigkeit. Hierbei handelt es sich insbesondere um ein ätherisches Öl. Diese Flüssigkeit wird auch als Inhalations- oder Inhalierflüssigkeit bezeichnet. Bei dem Speichermittel 3 selbst kann es sich um ein textiles Material, wie ein Vliesmaterial, handeln. Grundsätzlich können aber auch Baumwollmaterialien zum Einsatz kommen.

Das Speichermittel 3 hat im beschriebenen Ausführungsbeispiel ein Aufnahmevermögen für Inhalationsflüssigkeit von mindestens 3 g/g, insbesondere mindestens 4 g/g, vorzugsweise mindestens 5 g/g. Im in Fig. 2 dargestellten Ausführungsbeispiel hat das Speichermittel 3 eine Länge von 38 mm. Der Durchmesser kann je nach Art des Speichermittels 3 zwischen 1 und 20 mm, insbesondere zwischen 6 und 14 mm, bevorzugt zwischen 8 und 12 mm liegen.

Zur Aufnahme des Speichermittels 3 weist die Gehäusehülse 2 einen inneren Aufnahmeraum 4 auf, der stirnseitig durch die Stirnwandung 5 und umfangsmäßig durch die äußere Wandung 6 begrenzt ist.

Vorgesehen ist nun, dass innenseitig in der Gehäusehülse 2 wenigstens ein in radialer Richtung in den Aufnahmeraum 4 ragender Fixiervorsprung vorgesehen ist. Im dargestellten Ausführungsbeispiel sind in der Gehäusehülse 2 drei langgestreckte Rippen 7 vorgesehen, die zueinander gleich beabstandet sind und als Fixiervorsprünge dienen. Wie sich insbesondere aus Fig. 2 ergibt, liegt das Speichermittel 3 ausschließlich an den Rippen 7 an, ohne mit dem übrigen Innenwandungsbereich 8 der Wandung 6 in Kontakt zu kommen. Im Bereich der gemeinsamen Kontaktfläche zwischen den Rippen 7 und dem Speichermittel 3 ergibt sich eine reibschlüssige Fixierung des Speichermittels 3. Da zwischen dem Innenwandungsbereich 8 der Gehäusehülse 2 und dem Speichermittel 3 eine Berührungsfreiheit besteht, ist eine Diffusion der in dem Speichermittel 3 aufgenommenen Inhalationsflüssigkeit in diesem Bereich nicht möglich. Da die Rippen 7 im Übrigen baugleich ausgeführt sind, also jeweils gleich weit von der Wandung 6 abstehen, ergibt sich eine zentrierte Anordnung des Speichermittels 3 im Aufnahmeraum 4. Wie sich im Übrigen aus Fig. 2 ergibt, ist das Speichermittel 3 im gemeinsamen Kontaktbereich mit den Rippen 7 zusammengedrückt, was den Reibschluss zwischen den Rippen 7 und dem Speichermittel 3 begünstigt.

Die Gehäusehülse 2 selbst weist einen vorderen Hülsenbereich 9 und einen hinteren Hülsenbereich 10 auf. Der vordere Hülsenbereich 9 dient für den Nutzer als Inhalationsbereich. Dies bedeutet, dass der vordere Hülsenbereich 9 jedenfalls mit seinem vorderen Ende zum Einführen in bzw. Heranführen an die Nase des Nutzers vorgesehen ist. Hierzu weist der vordere Hülsenbereich 9 am vorderen Ende 11 eine in den Aufnahmeraum 4 führende mittige Nasenöffnung 12 auf. Konkret ist die Nasenöffnung 12 in der Stirnwandung 5 vorgesehen. Darüber hinaus befindet sich im Bereich des hinteren Endes des vorderen Hülsenbereichs 9 wenigstens eine Seitenöffnung 13. Bei der dargestellten Ausführungsform sind zwei Seitenöffnungen 13 vorgesehen, die sich auf gegenüberliegenden Seiten der Wandung 6 befinden. Die Seitenöffnungen 13 führen bei der dargestellten Ausführungsform radial in den Aufnahmeraum 4.

Wie sich aus verschiedenen Figuren ergibt, sind die Rippen 7 im Bereich des vorderen Endes 11 des vorderen Hülsenbereichs 9 vorgesehen. Letztlich erstrecken sich die Rippen 7 bei der dargestellten Ausführungsform von der Stirnwandung 5 aus in axialer Richtung in den Aufnahmeraum 4 hinein. Jede der Rippen 7 weist dabei jeweils einen Anschlag 14 zur Anlage der vorderen Stirnseite 15 des Speichermittels 3 auf. Weiterhin weist jede der Rippen 7 eine Auflaufschräge 16 auf, was das Einsetzen des Speichermittels 3 in die Gehäusehülse 2 begünstigt.

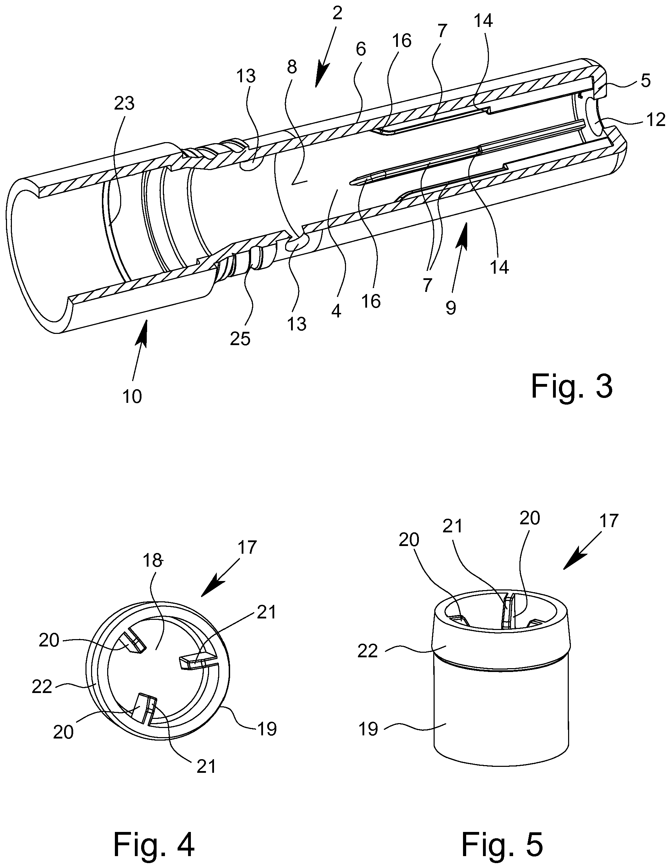

Zum Verschluss der Gehäusehülse 2 ist eine Stopfenhülse 17 vorgesehen, die in den hinteren Hülsenbereich 10 eingesetzt ist. Die Stopfenhülse 17, die ebenfalls aus Kunststoff besteht, ist für sich in den Fig. 4 und 5 dargestellt. Die Stopfenhülse 17 weist eine äußere Stirnwandung 18 und einen zylindrischen Hülsenbereich 19 auf. Innerhalb des zylindrischen Hülsenbereichs 19 befinden sich ebenfalls Fixiervorsprünge, die im dargestellten Ausführungsbeispiel wiederum als Rippen 20 ausgebildet sind. Die Rippen 20 weisen endseitig Auflaufanschläge 21 auf. Wie sich insbesondere aus Fig. 2 ergibt, ist das Speichermittel 3 im Endmontagezustand im Bereich der Auflaufanschläge 21 zusammengepresst.

Es versteht sich von selbst, dass statt der Rippen 7, 20 grundsätzlich auch andere Ausführungen von Fixiervorsprüngen vorgesehen sein können. Dies gilt insbesondere für die Rippen 20. Auch die Anzahl der jeweils vorgesehenen Rippen 7, 20, die im dargestellten Ausführungsbeispiel jeweils drei beträgt, kann variieren.

Zur Verbindung der Stopfenhülse 17 mit der Gehäusehülse 2 ist eine Verrastung vorgesehen. Hierzu ist außenseitig am zylindrischen Hülsenbereich 19 der Stopfenhülse 17 ein umlaufender Rastvorsprung 22 vorgesehen. Zu dem Rastvorsprung 22 korrespondiert innenseitig am hinteren Hülsenbereich 10 eine umlaufende Raststufe 23.

Zum Verschluss des vorderen Hülsenbereichs 9 mit der Nasenöffnung 12 und den Seitenöffnungen 13 ist eine Verschlusskappe 24 vorgesehen, die auf den vorderen Hülsenbereich 9 aufsetzbar ist. Im aufgesetzten Zustand werden über die Verschlusskappe 24 die Öffnungen 12, 13 abgedeckt und verschlossen. Im Übrigen ergibt sich im verschlossenen Zustand eine Luftdichtigkeit, so dass sich kein Austritt von Inhalationsflüssigkeit ergibt.

Im dargestellten Ausführungsbeispiel ist zur sicheren Halterung der Verschlusskappe 24 am vorderen Hülsenbereich 9 und zur Gewährleistung einer hinreichenden Dichtigkeit zwischen der Verschlusskappe 24 und dem Hülsengehäuse 2 eine Schraubverbindung vorgesehen. Hierzu befindet sich am Übergang vom vorderen Hülsenbereich 9 zum hinteren Hülsenbereich 10 ein Außengewinde 25, das mit einem Innengewinde 26 an der Verschlusskappe 24 korrespondiert. Die Schraubverbindung bzw. die Gewindesteigung der Schraubverbindung ist so ausgebildet, dass sich ein Öffnen der Verschlusskappe 24 bei einer Drehung der Verschlussklappe 24 von kleiner 360°, vorzugsweise zwischen 240° und 330° ergibt.

Im Übrigen versteht es sich, dass es grundsätzlich auch möglich ist, statt einer Schraubverbindung eine leicht lösbare Rastverbindung zwischen der Verschlusskappe 24 und der Gehäusehülse 2 vorzusehen.

Die Montage einschließlich der Befüllung des Inhalationsstiftes 1 erfolgt derart, dass zunächst das Speichermittel 3 in die Gehäusehülse 2 eingesetzt wird. Das Einführen des Speichermittels 3 in die Gehäusehülse 2 kann maximal so weit erfolgen, dass das Speichermittel 3 mit der vorderen Stirnseite 15 am Anschlag 14 anschlägt. In diesem Zustand ist das Speichermittel 3 unbeladen. Anschließend wird der Aufnahmeraum 4 durch Einsetzen der Stopfenhülse 17 in den hinteren Hülsenbereich 10 verschlossen. Dabei wird die Stopfenhülse 17 so weit in den äußeren Hülsenbereich 10 eingedrückt, bis eine Verrastung zwischen dem Rastvorsprung 22 und der Raststufe 23 erfolgt. Während des Einsetzens der Stopfenhülse 17 in den hinteren Hülsenbereich 10 kommen die Auflaufanschläge 21 mit den rückwärtigen Enden 27 des Speichermittels 3 in Kontakt. Dabei wird das Speichermittel 3 im noch immer unbeladenen Zustand im Kontaktbereich zusammengedrückt. Sollte sich das Speichermittel 3 nach dem vorläufigen Einsetzen noch nicht in Anlage mit den Anschlägen 14 befinden, so ergibt sich eine derartige Anlage spätestens zu diesem Zeitpunkt. Nach Verrastung der Stopfenhülse 17 ergibt eine Fixierung des Speichermittels 3 in axialer Richtung an sechs Stellen, nämlich an den drei Anschlagstellen 14 bzw. an den drei Stellen der Auflaufanschläge 21, wobei das Speichermittel 3 geringfügig eingespannt und damit fixiert ist.

Anschließend wird die mit dem Speichermittel 3 versehene und über die Stopfenhülse 17 geschlossene Gehäusehülse 2 mit der Inhalationsflüssigkeit beladen. Dies erfolgt über die Nasenöffnung 12 automatisch. Hierzu wird eine Befüllnadel in das zentralfixierte Speichermittel 3 eingebracht. Die Einbringung kann dabei derart erfolgen, dass die Befüllnadel bis in den unteren Bereich des Speichermittels 3 geführt wird und beim anschließenden Ab- oder Zurückziehen Inhalationsflüssigkeit kontinuierlich oder in Stufen abgegeben wird. Auf diese Weise kann eine optimale Beladung des Speichermittels 3 über dessen gesamte Länge gewährleistet werden. Es versteht sich, dass es grundsätzlich natürlich auch möglich ist, dass die Befüllnadel lediglich in den oberen Bereich des Speichermittels 3 eingesetzt und dann über eine vorgegebene Zeit Inhalationsflüssigkeit abgegeben wird, die anschließend von dem Speichermittel 3 aufgenommen wird.

Die zuvor beschriebenen Arten der Beladung des Speichermittels 3 haben sich als ausgesprochen gut erwiesen, da sich aufgrund der Beladung des Speichermittels 3 im bereits montierten Zustand eine sehr hygienische und sterile Situation ergibt. Darüber hinaus ist eine sehr exakte Dosierung der Inhalationsflüssigkeit möglich.

Im Rahmen der vorliegenden Erfindung hat es sich als vorteilhaft erwiesen, wenn das mindestens eine ätherische Öl auf Basis einer Zusammensetzung ausgebildet ist, welche zur Verwendung bei der prophylaktischen und/oder therapeutischen Behandlung von Erkrankungen geeignet ist, bzw. eine Zusammensetzung zur Verwendung bei der prophylaktischen bzw. therapeutischen Behandlung von Erkrankungen ist, wobei die Zusammensetzung mindestens zwei voneinander verschiedene ätherische Öle, insbesondere mindestens drei voneinander verschiedene ätherische Öle, aufweist, wobei die ätherischen Öle ausgewählt sind aus der Gruppe von Bergamotteöl (Citrus aurantium var. bergamia), Cedernholzöl (Cupressus funebris Endlicher), Cypressenöl (Cupressus sempervirens), Eukalyptusöl (Eucalyptus globulus), Geraniumöl (Pelargonium graveolens), Gingergrasöl (Cymbopogon martinii var. sofia), Lavendelöl (Lavandula angustifolia), Majoranöl (Origanum majorana), Mandarinenöl (Citrus reticulata), Muskatellersalbeiöl (Salvia sclarea), Orangenöl (Citrus aurantium var. dulcis), Pfefferminzöl (Mentha piperita), Pfefferöl (Piper nigrum), Rosmarinöl (Rosmarinus officinalis), Thymianöl (Thymus serpyllum), Vetiveröl (Vetiveria zizanoides), Wacholderbeeröl (Juniperus communis L.), Zimtrindenöl (Cinnamomum ceylanicum), Zitronenöl (Citrus limon), Grapefruitöl (Citrus decumana), Basilikumöl (Ocimum basilicum), Fichtenöl (Picea excelsa) sowie deren Mischungen und Kombinationen.

Gegenstand der vorliegenden Erfindung gemäß einer bevorzugten Ausführungsform ist somit insbesondere ein Inhalationsstift, wobei der Inhalationsstift 1 eine Zusammensetzung zur Verwendung bei der prophylaktischen und/oder therapeutischen Behandlung von Erkrankungen enthält, wobei der Inhalationsstift in seinem Inneren ein Speichermittel, vorzugsweise ein textiles Speichermittel, aufweist, wobei das Speichermittel mit der Zusammensetzung versehen und/oder beaufschlagt ist und
- wobei die Zusammensetzung mindestens zwei voneinander verschiedene ätherische Öle, insbesondere mindestens drei voneinander verschiedene ätherische Öle, aufweist, wobei die ätherischen Öle ausgewählt sind aus der Gruppe von
   Bergamotteöl (Citrus aurantium var. bergamia),
   Cedernholzöl (Cupressus funebris Endlicher),
   Cypressenöl (Cupressus sempervirens),
   Eukalyptusöl (Eucalyptus globulus),
   Geraniumöl (Pelargonium graveolens),
   Gingergrasöl (Cymbopogon martinii var. sofia),
   Lavendelöl (Lavandula angustifolia),
   Majoranöl (Origanum majorana),
   Mandarinenöl (Citrus reticulata),
   Muskatellersalbeiöl (Salvia sclarea),
   Orangenöl (Citrus aurantium var. dulcis),
   Pfefferminzöl (Mentha piperita),
   Pfefferöl (Piper nigrum),
   Rosmarinöl (Rosmarinus officinalis),
   Thymianöl (Thymus serpyllum),
   Vetiveröl (Vetiveria zizanoides),
   Wacholderbeeröl (Juniperus communis L.),
   Zimtrindenöl (Cinnamomum ceylanicum),
   Zitronenöl (Citrus limon),
   Grapefruitöl (Citrus decumana),
   Basilikumöl (Ocimum basilicum),
   Fichtenöl (Picea excelsa)
   sowie deren Mischungen und Kombinationen.

Der Begriff "Zusammensetzung", wie er im Rahmen der vorliegenden Erfindung verwendet wird, ist sehr umfassend zu verstehen und bezeichnet nicht nur Zusammensetzungen zu prophylaktischen bzw. therapeutischen Behandlungszwecken, insbesondere nicht nur Arzneimittel und Medikamente oder Medizinprodukte, sondern auch Lebensmittel, Nahrungsergänzungsmittel, Lebensmittelbestandteile oder -zusatzstoffe, Aromazusammensetzungen und homöopathische Mittel, insbesondere Lebensmittel und Lebensmittelbestandteile oder -zusatzstoffe.

Überraschenderweise konnten von der Anmelderin im Rahmen der vorliegenden Erfindung Konzepte bzw. Zusammensetzungen bereitgestellt werden, welche eine wirkungsvolle und zielgerichtete Behandlung verschiedenster Erkrankungen auf einem aromatherapeutischen Ansatz gewährleisten. Mit den Zusammensetzungen auf Basis zielgerichtet ausgewählter Kombinationen von ätherischen Ölen kann eine Vielzahl von Erkrankungen sowohl physiologischer als auch psychosomatischer Art behandelt werden. Im Rahmen der vorliegenden Erfindung ist es somit gelungen, anwendungsfertige und komfortabel applizierbare Komplex- bzw. Kombinationspräparate auf Basis von ätherischen Ölen zur gezielten aromatherapiebasierten Behandlung ausgewählter Erkrankungen bereitzustellen. Die medizinische Verwendung dieser Öle ist nicht Teil der Erfindung.

Vorteilhafterweise zeichnen sich die Konzepte zur Applikation der Zusammensetzungen dadurch aus, dass keine bzw. zumindest im Wesentlichen keine Nebenwirkungen auftreten, aber dennoch eine hervorragende Wirkeffizienz gewährleistet ist. Durch den Inhalationsstift können die Zusammensetzungen in hoher Konzentration sehr nah an die Nase bzw. die Rezeptoren der Nasenschleimhaut, insbesondere den Wirkort, gebracht werden, ohne dass es jedoch zu einer direkten Berührung der Haut bzw. Schleimhaut mit den ätherischen Ölen kommt. Allergische Reaktionen und Hautreizungen können auf diese Weise vermieden werden. Weiterhin besteht aufgrund der Verwendung eines Inhalationsstifts nicht das Problem, welches beispielsweise bei einem Einsatz von Duftlampen auftritt, nämlich dass die Wirk- bzw. Duftstoffe der ätherischen Öle im ganzen Raum verteilt werden. Weiterhin wird durch die zielgerichtete Anwendung der Zusammensetzung eine Habituation bzw. Gewöhnung an den applizierten Geruch, welche mit einem Verlust der Wirksamkeit einhergehen würde, verhindert, wie es bei Applikation der Aromatherapie über die Raumluft häufig der Fall ist.

Was die Anwendung der Zusammensetzung anbelangt, so resultiert die hervorragende Wirkeffizienz daraus, dass es - ohne sich hierbei auf diese Theorie beschränken zu wollen - bei dem bewussten und tiefen Einatmen der ätherischen Öle bzw. der volatilen Bestandteile der ätherischen Öle zu einer zielgerichteten Bindung der Duftmoleküle bzw. Duftstoffe an die Geruchsrezeptoren der Riechschleimhaut in der Nase kommt. Über sensorische Nerven, welche über Öffnungen in der Cribriform-Platte in Verbindung mit den Rezeptoren stehen, werden die durch die Bindung bzw. Anlagerung der Duftstoffe bzw. Duftmoleküle resultierenden olfaktorischen Signale an das Gehirn weitergeleitet, was wiederum in einer Entfaltung der aromatherapeutischen Wirkung resultiert.

Im Rahmen der vorliegenden Erfindung werden unter ätherischen Ölen hochkonzentrierte Pflanzenessenzen bzw. Pflanzenextrakte verstanden, welche mittels Wasserdampfdestillation, Schalenpressung oder Extraktion gewonnen werden können. Ätherische Öle zeichnen sich insbesondere durch ihre volatilen Eigenschaften aus, was auch die hervorragende Eignung zur Aromatherapie begründet. Insgesamt existiert eine Vielzahl von ätherischen Ölen auf Basis verschiedenster Pflanzenarten, welche dementsprechend vielseitige Eigenschaften und Anwendungsmöglichkeiten bieten. Je nach Öl können ätherische Öle bis zu 400 verschiedene Wirkstoffe auf Basis von sekundären Pflanzenstoffen enthalten. Bei den sekundären Pflanzenstoffen handelt es sich vorwiegend um Gemische verschiedener Terpene, Sesquiterpene und aromatischer Verbindungen. Terpene basieren auf Isopreneinheiten, wobei Monoterpene aus zwei und Sesquiterpene aus drei Isopreneinheiten bestehen. Insgesamt ist das Wirkspektrum ätherischer Öle breit gefächert, so dass verschiedenste Wirkungen erzielt werden können.

Im Rahmen der vorliegenden Erfindung wurde überraschend gefunden, dass sich bestimmte Kombinationen von ätherischen Ölen auf Basis der obigen zweckgerichteten Auswahl in ihrer Wirkung derart ergänzen bzw. verstärken, dass bestimmte Erkrankungen bzw. Krankheitsbilder mit Komplexpräparaten bzw. Kombinationspräparaten äußerst zielgerichtet und wirkungsvoll behandelt werden können, ohne dass Nebenwirkungen auftreten, wie es häufig bei Medikamenten der Schulmedizin der Fall ist. Durch die Applikation der Zusammensetzungen unter Verwendung der erfindungsgemäßen Inhalationsstifte kann die Wirkung zudem weitergehend gesteigert werden, da die Bindung der Wirkstoffe an die Rezeptoren der Nasenschleimhaut optimiert wird. Die medizinische Verwendung dieser Zusammensetzungen ist nicht Teil der Erfindung. Der Inhalationsstift zur Applikation der Zusammensetzung kann in vielfältiger Art und Weise ausgestaltet sein. Bevorzugte Ausführungsformen sind nachfolgend zum besseren Verständnis im Detail beschrieben.

In Bezug auf die Applikation der Zusammensetzung, insbesondere hinsichtlich der Aufnahme und Freisetzung der Wirkstoffe der ätherischen Öle, hat es sich als besonders vorteilhaft erwiesen, wenn das Speichermittel des Inhalationsstifts zur Aufnahme der Zusammensetzung porös bzw. luftdurchlässig ausgebildet ist.

In diesem Zusammenhang ist es insbesondere vorgesehen, dass das Speichermittel ein textiles Material umfasst bzw. hieraus gebildet ist, insbesondere auf Basis von Baumwolle, Cellulose oder Cellulosederivaten, vorzugsweise Celluloseacetaten (wie z. B. Speichermittel auf Basis von *Bonded Acetate Fibre(s)*)*,* Glycerintriacetaten sowie deren Mischungen und Kombinationen. Die vorgenannten Materialien sowie die Porosität bzw. Luftdurchlässigkeit des Speichermittels erlauben eine besonders effiziente Aufnahme der Zusammensetzung in das Speichermittel, wobei insbesondere auch ein vorzeitiges Verdunsten verhindert werden kann. Durch die Materialauswahl kann insbesondere auch die Haltbarkeit der Zusammensetzungen bzw. der anwendungsfertigen Inhalationsstifte verbessert werden.

Darüber hinaus ist es erfindungsgemäß bevorzugt, wenn das Speichermittel ein Wasseraufnahmevermögen von mindestens 3 g/g, insbesondere mindestens 4 g/g, vorzugsweise mindestens 5 g/g, aufweist (Menge bzw. Gewicht Wasser pro Menge bzw. Gewicht Speichermittel). Erfindungsgemäß hat es sich als besonders vorteilhaft erwiesen, wenn das Speichermittel eine erhöhte Wasseraufnahmekapazität aufweist, so dass große Mengen der Zusammensetzung in das Speichermittel eingebracht werden können, was die Haltbarkeit bzw. Nachhaltigkeit der anwendungsfertigen Inhalationsstifte wiederum weiter verbessert.

Zudem ist es erfindungsgemäß insbesondere vorgesehen, dass das Speichermittel zylinderförmig oder stabförmig ausgebildet ist, insbesondere mit Längen von 10 bis 100 mm bzw. mit Dicken (Durchmessern) von 1 bis 20 mm.

In Bezug auf das Speichermittel ist es zudem bevorzugt, wenn dieses ein Gewicht von 0,1 bis 10,0 g, insbesondere 0,2 bis 7,5 g, vorzugsweise 0,3 bis 5 g, aufweist. Dabei beziehen sich die Gewichtsangaben insbesondere auf den unbeladenen Zustand des Speichermittels.

Was die Menge der Zusammensetzung in dem Inhalationsstift bzw. dem Speichermittel anbelangt, so kann diese in weiten Bereichen variieren. Erfindungsgemäß bevorzugt ist es jedoch, wenn der Inhalationsstift bzw. das Speichermittel die Zusammensetzung in wirksamen, insbesondere therapeutisch wirksamen Mengen enthält bzw. enthalten.

Eine besonders gute Wirksamkeit insbesondere bezüglich der Freisetzung der ätherischen Öle wird insbesondere gewährleistet, wenn der Inhalationsstift und/oder das Speichermittel die Zusammensetzung in Mengen von 0,01 bis 10,0 ml, insbesondere 0,1 bis 5 ml, vorzugsweise 0,2 bis 1 ml, enthält bzw. enthalten.

Gleichermaßen kann es vorgesehen sein, dass der Inhalationsstift bzw. das Speichermittel die Zusammensetzung in Mengen von 0,005 bis 15 g, insbesondere 0,05 bis 7,5 g, vorzugsweise 0,01 bis 1,5 g, besonders bevorzugt 0,1 bis 1 g, enthält bzw. enthalten. Auch kann es im Rahmen der vorliegenden Erfindung vorgesehen sein, dass der Inhalationsstift und/oder das Speichermittel die Zusammensetzung in relativen Mengen von 0,1 bis 300 Gew.-%, insbesondere 0,2 bis 150 Gew.-%, vorzugsweise 0,5 bis 100 Gew.-%, besonders bevorzugt 1 bis 80 Gew.-%, bezogen auf das Speichermittel, enthält bzw. enthalten.

Was die Anwendung der Zusammensetzung anbelangt, so ist es üblicherweise im Rahmen der vorliegenden Erfindung vorgesehen, dass das Speichermittel bei Applikation, insbesondere inhalativer Applikation, die Zusammensetzung in wirksamen, insbesondere therapeutisch wirksamen Mengen freizusetzen imstande ist. Mit anderen Worten ist es erfindungsgemäß vorgesehen, dass die Zusammensetzung nasal appliziert wird, d. h. der Inhalationsstift direkt vor die Nase gehalten wird bzw. mit der Spitze in die Nase eingeführt wird. Auf diese Weise kann - wie nachfolgend noch im Detail erläutert - eine unkomplizierte und zumindest im Wesentlichen nebenwirkungsfreie Behandlung verschiedenster Erkrankungen gewährleistet werden, da die ätherischen Öle nicht in Kontakt mit der Haut bzw. Schleimhaut kommen. Darüber hinaus wird die Wirksamkeit gegenüber den bekannten Methoden zur Aromatherapie verbessert, da die Wirkstoffe direkt in die Nähe der Rezeptoren der Nasenschleimhaut gebracht werden, jedoch ohne dass es zu einem direkten Kontakt von ätherischem Öl und Nasenschleimhaut, was wiederum zu (Schleim)Hautreizungen führen würde. Die Zusammensetzung nach der Erfindung kann somit nasal bzw. intranasal appliziert werden.

Was die zu behandelnden Erkrankungen im Speziellen anbelangt, hat sich im Rahmen der vorliegenden Erfindung gezeigt, dass mit zielgerichtet ausgewählten Kombinationen von ätherischen Ölen auf Basis der oben genannten Auswahl eine besonders effiziente Behandlung von Erkrankungen möglich ist, welche ausgewählt sind aus kardiovaskulären Erkrankungen (Erkrankungen des Herz/KreislaufSystems), insbesondere Bluthochdruck (Hypertonie); Infekten, insbesondere Erkältungen, Rhinitis und Bronchitis; Stoffwechselerkrankungen, insbesondere Diabetes oder Übergewicht; psychosomatischen Erkrankungen, insbesondere Stress, physisch-psychischen Überlastungen, Entzugssyndromen und Schlafstörungen; und/oder Schmerzzuständen.

Bei den vorgenannten Erkrankungen handelt es sich insbesondere auch um sogenannte "Volkskrankheiten", welche besonders häufig in der Bevölkerung auftreten und ein dementsprechender Behandlungs- bzw. Therapiebedarf besteht. Im Rahmen der vorliegenden Erfindung war es vollkommen überraschend, dass die Symptome der vorgenannten insbesondere systemischen Erkrankungen auf Basis der insbesondere nasalen Applikation der Zusammensetzungen signifikant gelindert werden können, ohne dass nennenswerte Nebenwirkungen auftreten. In diesem Zusammenhang können sowohl physische Symptome, wie ein zu hoher Blutdruck, Schmerzen, Herzrasen, Entzündungsanzeichen und dergleichen, als auch psychische bzw. psychosomatische Beschwerden, wie Abgeschlagenheit, Nervosität, Ruhelosigkeit, Anspannung und dergleichen, behandelt werden.

Insbesondere war es im Rahmen der vorliegenden Erfindung überraschend, dass sich durch Anwendung der Zusammensetzungen die Pharmakodynamik positiv beeinflussen bzw. beschleunigen lässt. Überraschenderweise hat sich gezeigt, dass bei Einsatz der zuvor beschriebenen Zusammensetzungen als Begleitmaßnahme, z. B. zur Schmerztherapie, die Latenz (d. h. die Zeit bis zum Wirkeintritt beispielsweise einer Schmerztablette) und die Zeit bis zur maximal erreichbaren Beschwerdelinderung (z. B. die Zeit bis zur völligen bzw. maximalen Schmerzfreiheit) verkürzt werden können.

In diesem Zusammenhang wird bereits auf die von der Anmelderin durchgeführten Wirksamkeitsstudien verwiesen, welche die überraschende Wirkeffizienz der Zusammensetzungen in Bezug auf bestimmte Erkrankungen eindrucksvoll belegen. Die medizinische Verwendung ist nicht Teil der Erfindung. Die hier offenbarten Zusammensetzungen sind in Verbindung mit dem Inhalationsstift gemäß Anspruch 1 Teil der Erfindung. Was die physikochemischen Parameter der Zusammensetzungen anbelangt, so sind diese nachfolgend im Detail beschrieben. Die Bestimmung der Parameter erfolgt anhand von DIN-genormten Messmethoden und Verfahren, welche dem Fachmann an sich bekannt sind und somit an dieser Stelle keinen weiteren Ausführungen bedürfen.

In Bezug auf die Dichte der Zusammensetzung, so liegt diese bei 20 °C und Atmosphärendruck (101.325 Pascal) vorzugsweise im Bereich von 0,5 bis 1,5 g/cm³, insbesondere im Bereich von 0,6 bis 1,2 g/cm³, vorzugsweise im Bereich von 0,7 bis 1,1 g/cm³.

Darüber hinaus ist es erfindungsgemäß vorgesehen, dass die Zusammensetzung bei 20 °C und Atmosphärendruck (101.325 Pascal) flüssig ist bzw. im flüssigen Aggregatzustand vorliegt.

Weiterhin weist die Zusammensetzung bei 20 °C üblicherweise einen Brechungsindex im Bereich von 1,300 bis 1,700, insbesondere im Bereich von 1,350 bis 1,650, vorzugsweise im Bereich von 1,400 bis 1,600, auf.

Zudem zeichnet sich die Zusammensetzung durch einen äußerst geringen Anteil an volatilen organischen Verbindungen aus. So ist es erfindungsgemäß vorgesehen, dass die Zusammensetzung einen Anteil an VOC (Volatile Organic Compounds) von weniger als 1 Gew.-%, insbesondere von weniger als 0,5 Gew.-%, vorzugsweise von 0 Gew.-%, bezogen auf die Zusammensetzung, aufweist. Die Zusammensetzung ist somit zumindest im Wesentlichen frei von schädlichen organischen Substanzen, so dass keine damit verbundenen Nebenwirkungen auftreten.

Was die Gewinnung der Zusammensetzung anbelangt, so erfolgt diese mittels der für ätherische Öle bekannten Herstellungsverfahren. Erfindungsgemäß ist es insbesondere vorgesehen, dass die Zusammensetzung mittels Pressung und/oder Wasserdampfdestillation, insbesondere aus den entsprechenden Ausgangsdrogen und/oder Pflanzenteilen, wie Fruchtschalen, Früchten, Holz, Blättern, Zweigen, Zweigspitzen, Blüten, Blütenständen, Gras, Kraut, Wurzeln, Rinden oder dergleichen, erhalten ist.

Weiterhin ist es vorgesehen, dass die Zusammensetzung volatil bzw. flüchtig ist. In diesem Zusammenhang ist es besonders bevorzugt, wenn die Zusammensetzung bei 20 °C einen Dampfdruck oberhalb von 20 mbar, insbesondere oberhalb von 24 mbar, vorzugsweise oberhalb von 28 mbar, bevorzugt oberhalb von 30 mbar, besonders bevorzugt oberhalb von 40 mbar, ganz besonders bevorzugt oberhalb von 60 mbar, noch mehr bevorzugt oberhalb von 100 mbar, aufweist. Die Wirkweise der Zusammensetzung begründet sich entsprechend in den volatilen bzw. flüchtigen Eigenschaften, da erst durch einen entsprechend hohen Dampfdruck die aromatherapeutische Wirkung entsteht bzw. die flüchtigen Duftstoffe nach Applikation bzw. Inhalation an ihre Wirkorte gelangen können.

Erfindungsgemäß hat es sich als besonders vorteilhaft erwiesen, wenn die Zusammensetzung mindestens eine der nachfolgenden Kombinationen von ätherischen Ölen umfasst:
(i) Pfefferminzöl/Rosmarinöl/Lavendelöl, insbesondere in gewichtsbezogenen Verhältnissen von 40-80/10-50/1-20, vorzugsweise zur prophylaktischen und/oder therapeutischen Behandlung von Schmerzzuständen;
(ii) Grapefruitöl/Pfefferminzöl, insbesondere in gewichtsbezogenen Verhältnissen von 30-70/30-70, vorzugsweise zur prophylaktischen und/oder therapeutischen Behandlung von Stoffwechselerkrankungen, insbesondere Diabetes oder Übergewicht;
(iii) Eukalyptusöl/Pfefferminzöl/Thymianöl, insbesondere in gewichtsbezogenen Verhältnissen von 60-90/5-20/5-20, vorzugsweise zur prophylaktischen und/oder therapeutischen Behandlung von Infekten, insbesondere Erkältungen, Rhinitis und Bronchitis;
(iv) Lavendelöl/Vetiveröl/Mandarinenöl, insbesondere in gewichtsbezogenen Verhältnissen von 20-50/20-50/15-40, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(v) Zitronenöl/Pfefferminzöl/Rosmarinöl/Grapefruitöl/Pfefferöl/Basilikumöl, insbesondere in gewichtsbezogenen Verhältnissen von 15-30/15-30/15-30/10-25/2-10/1-8;
(vi) Bergamotteöl/Zitronenöl/Cedernholzöl/Muskatellersalbeiöl/Vetiveröl, insbesondere in gewichtsbezogenen Verhältnissen von 25-40/20-35/10-20/10-20/5-20, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(vii) Pfefferminzöl/Vetiveröl/Wacholderbeeröl/Thymianöl, insbesondere in gewichtsbezogenen Verhältnissen von 25-45/20-40/20-40/1-15;
(viii) Pfefferminzöl/Cypressenöl/Geraniumöl/Gingergrasöl, insbesondere in gewichtsbezogenen Verhältnissen von 40-60/25-45/10-30/1-10;
(ix) Pfefferminzöl/Rosmarinöl/Zimtrindenöl, insbesondere in gewichtsbezogenen Verhältnissen von 65-95/1-15/1-10, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(x) Pfefferminzöl/Grapefruitöl/Rosmarinöl, insbesondere in gewichtsbezogenen Verhältnissen von 75-95/1-12/1-5;
(xi) Bergamotteöl/Majoranöl/Lavendelöl/Orangenöl, insbesondere in gewichtsbezogenen Verhältnissen von 30-50/10-30/10-30/10-30;
(xii) Rosmarinöl/Pfefferminzöl/Basilikumöl, insbesondere in gewichtsbezogenen Verhältnissen von 40-60/35-55/1-15.

In diesem Zusammenhang wird auch auf die nachfolgenden Ausführungsbeispiele verwiesen, welche die Wirkeffizienz der vorgenannten Wirkstoffkombinationen eindrucksvoll belegen. Es werden somit Konzepte bzw. Zusammensetzungen bereitgestellt, welche in völlig überraschender Weise eine zielgerichtete und wirkeffiziente Behandlung von verschiedenen Erkrankungen auf Basis von Aromatherapien ermöglichen. Die nasale Applikation der Zusammensetzungen ist besonders einfach in der Handhabung und zeichnet sich darüber hinaus durch ihre äußerst geringen Nebenwirkungen aus. Die hervorragende Wirksamkeit der Zusammensetzung bei der Behandlung der oben genannten Erkrankungen wird auch auf Basis der nachfolgend geschilderten, von der Anmelderin durchgeführten Ausführungsbeispiele bzw. Anwendungsstudien in eindrucksvoller Weise belegt.

Gegenstand der vorliegenden Erfindung ist somit ein Inhalationsstift für die inhalative Applikation von Wirkstoffen, wobei der Inhalationsstift in seinem Inneren ein Speichermittel, vorzugsweise ein textiles Speichermittel, aufweist, wobei das Speichermittel mit einer Zusammensetzung zur prophylaktischen und/oder therapeutischen Behandlung von Erkrankungen versehen und/oder beaufschlagt ist, wobei die Zusammensetzung mindestens zwei voneinander verschiedene ätherische Öle, insbesondere mindestens drei voneinander verschiedene ätherische Öle, aufweist, wobei die ätherischen Öle ausgewählt sind aus der Gruppe von Bergamotteöl (Citrus aurantium var. bergamia), Cedernholzöl (Cupressus funebris Endlicher), Cypressenöl (Cupressus sempervirens), Eukalyptusöl (Eucalyptus globulus), Geraniumöl (Pelargonium graveolens), Gingergrasöl (Cymbopogon martinii var. sofia), Lavendelöl (Lavandula angustifolia), Majoranöl (Origanum majorana), Mandarinenöl (Citrus reticulata), Muskatellersalbeiöl (Salvia sclarea), Orangenöl (Citrus aurantium var. dulcis), Pfefferminzöl (Mentha piperita), Pfefferöl (Piper nigrum), Rosmarinöl (Rosmarinus officinalis), Thymianöl (Thymus serpyllum), Vetiveröl (Vetiveria zizanoides), Wacholderbeeröl (Juniperus communis L.), Zimtrindenöl (Cinnamomum ceylanicum), Zitronenöl (Citrus limon), Grapefruitöl (Citrus decumana), Basilikumöl (Ocimum basilicum), Fichtenöl (Picea excelsa) sowie deren Mischungen und Kombinationen.

Überraschenderweise wurde von der Anmelderin gefunden, dass mit dem erfindungsgemäßen Inhalationsstift die Wirksamkeit von Wirkstoffen auf Basis von ätherischen Ölen signifikant verbessert werden kann. Ohne sich hierbei auf diese Theorie beschränken zu wollen, verbessert die Anwendung des erfindungsgemäßen Inhalationsstifts die Applikation bzw. Verabreichung der Zusammensetzung insbesondere auch auf Basis einer strömungstechnischen Optimierung des Inhalationsstifts mit resultierender verbesserter Abgabe der Zusammensetzung, was folglich auch die Bindung der Wirk- bzw. Duftstoffe an die Rezeptoren in der Nasenschleimhaut verbessert, insbesondere im Hinblick auf eine optimierte Konzentration der Wirkkomponenten am Wirkort. Darüber hinaus wird ein vorzeitiges Verdunsten der Wirkstoffe aus dem Inhalationsstift bzw. in das Material des Inhalationsstiftes vermieden. Darüber hinaus ist der erfindungsgemäße Inhalationsstift äußerst komfortabel in der Handhabung und kann jederzeit vom Patienten mitgeführt werden, da er nur eine geringe Größe besitzt. Im Gegensatz zu den bekannten Methoden bzw. Maßnahmen zur Aromatherapie, wie beispielsweise Duftlampen oder Aromaölmassagen, kann der erfindungsgemäße Inhalationsstift auch ortsunabhängig appliziert werden.

Darüber hinaus betrifft die vorliegende Erfindung - gemäß einem **weiteren** Aspekt der vorliegenden Erfindung - ein Verfahren zur Montage eines Inhalationsstiftes der vorgenannten Art, wobei das Speichermittel zunächst in den Aufnahmeraum eingesetzt wird, der Aufnahmeraum anschließend geschlossen wird und die Inhalierflüssigkeit nach dem Schließen des Aufnahmeraums über die Nasenöffnung in das Speichermittel eingebracht wird.

Wie zuvor bereits ausgeführt worden ist, lässt sich der Inhalationsstift auf die vorstehende Art und Weise sehr einfach und kostengünstig mit einer genau vorgegebenen, definierten Dosierung beladen, wobei die Beladung letztlich an Ort und Stelle erfolgt, so dass eine Sterilitätsproblematik nicht auftritt.

Im Zusammenhang mit der Befüllung des Inhalationsstifts hat es sich zudem als vorteilhaft erwiesen, wenn zumindest während des Befüllungsprozesses bzw. während des Einbringens der Inhalierflüssigkeit in das Speichermittel, insbesondere zumindest in der Hülse ein Unterdruck bzw. ein Vakuum angelegt wird. Die eingebrachte Wirkstofflösung (Inhalierlösung), insbesondere die vorzugsweise volatile Flüssigkeit bzw. die Zusammensetzung nach der vorliegenden Erfindung, muss dann nicht erst die sich in der Hülse befindliche Luft verdrängen, so dass die Befüllungs- bzw. Produktionseffizienz insgesamt gesteigert werden kann.

Weiterhin kann es vorgesehen sein, dass das Speichermittel im unbeladenen Zustand zunächst in den Aufnahmeraum eingesetzt wird, dass der Aufnahmeraum anschließend durch Einsetzen der Stopfenhülse in den äußeren Hülsenbereich geschlossen wird und dass das Speichermedium in seiner endgültigen Montagelage aufgrund der zuvor eingeschobenen Stopfenhülse an beiden Enden in Längsrichtung fixiert wird. Dabei sind letztlich die Abmaße der Länge des Speichermittels einerseits und/oder des lichten Abstands zwischen den Fixierpunkten der Gehäusehülse und der Stopfenhülse andererseits so, dass sich entweder eine Spielfreiheit, d. h. eine unmittelbare Anlage, ergibt, oder aber dass das Speichermedium an seinen Enden mit geringem Druck eingespannt ist. Im Übrigen ermöglicht es diese Art der Montage, dass die Endmontage des Speichermediums letztlich über das Einschieben der Stopfenhülse erfolgt. Auf diese Weise ist im Ergebnis eine definierte Anordnung des Speichermittels in der Gehäusehülse in jedem Falle gewährleistet.

Für weitergehende Einzelheiten zu diesem Erfindungsaspekt kann - zur Vermeidung unnötiger Wiederholungen - auf obige Ausführungen zu dem ersten Erfindungsaspekt verwiesen werden, welche in Bezug auf das erfindungsgemäße Verfahren entsprechend gelten.

Die in den erfindungsgemäßen Inhalationsstift eingebrachten Zusammensetzungen sind somit insgesamt zur Verwendung bei der nichttherapeutischen, insbesondere symptomatischen Anwendung, geeignet, insbesondere zur Anwendung als Lebensmittel, Lebensmittelbestandteil oder -zusatzstoff, Nahrungsergänzungsmittel, Aromazusammensetzung oder dergleichen, bei Erkrankungen aus der Gruppe von kardiovaskulären Erkrankungen (Erkrankungen des Herz/Kreislauf-Systems), insbesondere Bluthochdruck (Hypertonie); Infekten, insbesondere Erkältungen, Rhinitis und Bronchitis; Stoffwechselerkrankungen, insbesondere Diabetes oder Übergewicht; psychosomatischen Erkrankungen, insbesondere Stress, physisch-psychischen Überlastungen, Entzugssyndromen und Schlafstörungen; und/oder Schmerzzuständen.

Eine nichttherapeutische, insbesondere symptomatische Anwendung bezeichnet insbesondere eine Verwendung der zuvor beschriebenen Zusammensetzung welche bei einer unter einer der vorgenannten Erkrankungen leidenden Person nicht auf eine Heilung oder Linderung im medizinischen oder therapeutischen Sinne abzielt, aber dennoch auf eine zumindest subjektive Verbesserung des Wohlbefindens und/oder des psychischen Zustands der betreffenden Person abzielt. Zu diesem Zweck kann die eingesetzte Zusammensetzung als Lebensmittel, Lebensmittelbestandteil oder -zusatzstoff, Nahrungsergänzungsmittel, Aromazusammensetzung oder dergleichen vorliegen bzw. eingesetzt werden, und zwar mittels des zuvor beschriebenen Inhalationsstifts.

Die vorliegende Erfindung wird anhand der folgenden Ausführungsbeispiele veranschaulicht, welche die vorliegende Erfindung jedoch keinesfalls beschränken.

### Bezugszeichen

- 1: Inhalationsstift
- 2: Gehäusehülse
- 3: Speichermittel
- 4: Aufnahmeraum
- 5: Stirnwandung
- 6: Wandung
- 7: Rippe
- 8: Innenwandungsbereich
- 9: vorderer Hülsenbereich
- 10: hinterer Hülsenbereich
- 11: vorderes Ende
- 12: Nasenöffnung
- 13: Seitenöffnung
- 14: Anschlag
- 15: Stirnseite
- 16: Auflaufschräge
- 17: Stopfenhülse
- 18: Stirnwandung
- 19: zylindrischer Hülsenbereich
- 20: Rippe
- 21: Auflaufschräge
- 22: Rastvorsprung
- 23: Raststufe
- 24: Verschlusskappe
- 25: Außengewinde
- 26: Innengewinde
- 27: rückwärtiges Ende

### Ausführungsbeispiele:

Im Rahmen der von der Anmelderin durchgeführten Anwendungs- und Wirksamkeitsstudien wurde der Einfluss verschiedener erfindungsgemäßer Inhalationsstifte in Bezug auf unterschiedliche medizinische bzw. psychosomatische Indikationen untersucht.

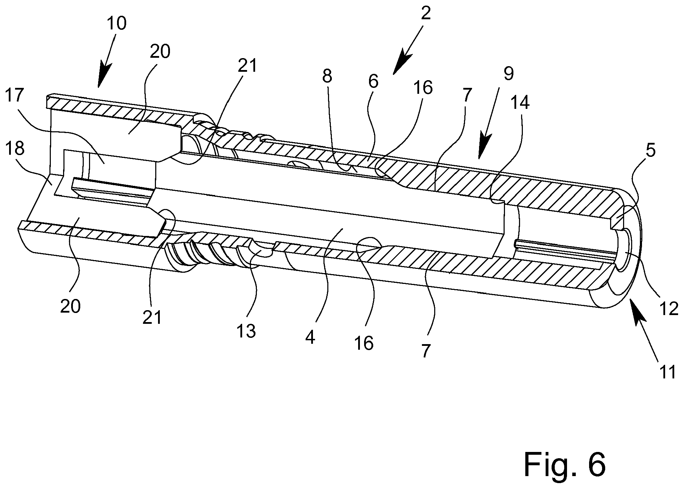

Zur Durchführung der nachfolgenden Anwendungsstudien wurde ein erfindungsgemäßer Inhalationsstift verwendet, wie er zuvor beschrieben wurde und insbesondere auch in den Fig. 1 bis 6 abgebildet ist.

Als Speichermittel für die jeweils eingesetzte Zusammensetzung wurden Cellulosefilter bzw. Celluloseacetatfilter mit einer Größe von 38 mm Länge und 8 mm Dicke verwendet, welche über die Fa. IVF Hartmann AG, Neuhausen, Schweiz bzw. die Fa. Filtrona Porous Technologies, Yverdon-les-bains, Schweiz, erhältlich sind. Das Speichermittel in den erfindungsgemäßen Inhalationsstiften wurde gemäß dem diesbezüglichen erfindungsgemäßen Verfahren mit jeweils 0,8 ml der Zusammensetzungen beschickt.

### 1. Inhalationsstift mit einer Aromaölkombination von Lavendelöl, Vetiveröl und Mandarinenöl

Der erfindungsgemäße Inhalationsstift enthielt zu Testzwecken 0,8 ml einer Zusammensetzung, welche 35 Gew.-% Lavendelöl, 35 Gew.-% Vetiveröl sowie 30 Gew.-% Mandarinenöl, jeweils bezogen auf die Wirkstoffzusammensetzung, enthielt. Im Rahmen der von der Anmelderin durchgeführten Anwendungsstudien wurde der Einfluss dieser erfindungsgemäßen Kombination von ätherischen Ölen auf das Herz/Kreislauf-System von Personen, welche unter chronischem Stress litten, untersucht. Als Vergleich diente ein erfindungsgemäßer Inhalationsstift ohne ätherische Öle ("Placebostift"). Darüber hinaus wurde zu Vergleichszwecken ermittelt, wie stark der Einfluss der wissenschaftlich anerkannten Entspannungstechnik der Progressiven Muskelrelaxation nach Jacobson auf das Herz/Kreislauf-System ist.

Dazu wurde eine Probandengruppe von 90 Personen, von denen 66 weiblich und 24 männlich waren, mit einem Alter im Bereich von 18 bis 65 Jahren herangezogen. Die Probanden wurden unter der Voraussetzung zu der Studie zugelassen, dass bei ihnen das Kriterium eines erhöhten chronischen Stresslevels erfüllt ist. Um die erhaltenen Ergebnisse nicht zu verfälschen, wurden medizinisch-klinische Diagnosen, wie Blutdruckerkrankungen, kardiovaskuläre Erkrankungen, Diabetes, Drogen- oder Medikamentenabhängigkeit, epileptische Anfälle, Schwangerschaft, psychische Erkrankungen und akute Erkältungen, bei den Probanden ausgeschlossen.

Das Studiendesign basierte auf einer placebokontrollierten, randomisierten, dreiarmigen Explorationsstudie mit Messwiederholung. Die Probanden wurden auf die nachfolgend in Tabelle 1 aufgeführten Vergleichsgruppen von jeweils 30 Probanden aufgeteilt:

**Tabelle 1: Experimentalbedingungen, Instruktionen und getestete Wirkungen**

| **Gruppe** | **Instruktion** | **Getestete Wirkung** |
|---|---|---|
| Kontroll-/Placebogruppe | Inhalationsstift ohne Wirkstoff | Ausschluss unspezifischer Effekte (Gewöhnung, Entspannung) |
| Progressive Muskelrelaxation (PMR) nach Jacobson | Wissenschaftlich anerkanntes Beruhigungs- und Entspannungsverfahren | Vergleich der erzielten physiologischen und psychologischen Entspannung bei der Indikation von chronischem Stress |
| Inhalationsstift | Aromatherapie auf Basis der Wirkstoffkombination Lavendelöl, Vetiveröl und Mandarinenöl | Untersuchung der Duftstoffwirkung in Bezug auf die physiologische und psychologische Entspannung bei der Indikation von chronischem Stress |

### Studienablauf

Die Probanden wurden jeweils einzeln getestet, wobei die Studiendauer insgesamt 45 min betrug. Die Studie begann mit dem Empfang der Probanden zum Zeitpunkt t₀.

Nach 12 min zum Zeitpunkt t₁₂ beurteilten die Probanden ihre aktuelle Befindlichkeit bzw. ihr Wohlbefinden anhand eines auszufüllenden Fragebogens. Der Fragebogen enthielt 20 die Befindlichkeit bzw. das Wohlbefinden betreffende Adjektive, umfassend sowohl positive als auch negative Affekte, wobei die Probanden auf einer vierstufigen Skala beurteilten, ob die jeweiligen Adjektive "gar nicht", "kaum", "etwas" oder "sehr" zutrafen. Im Rahmen der Beurteilung konnten Werte im Bereich von 20 bis 80 erzielt werden, wobei "80" dem positivsten Wohlbefinden und "20" dem negativsten Wohlbefinden entsprach.

Anschließend wurden nach 15 bis 20 min der Blutdruck sowie die Herzrate mit Hilfe von automatischen oszillometrischen Oberarmmessgeräten (Fa. Hartmann, Heidenheim, Deutschland) bestimmt.

25 min nach Beginn der Studie wurde zum Zeitpunkt t₂₅₋₃₅ die 10-minütige Intervention durchgeführt. Wie nachfolgend geschildert, unterschied sich die Intervention je nach Probandengruppe:

### a) Kontrollgruppe (Vergleich)

Die Intervention in der Kontrollgruppe bestand darin, dass die Probanden Placebo-Inhalationsstifte erhielten, welche bezüglich ihrer Form und Größe mit den erfindungsgemäßen Inhalationsstiften identisch waren, jedoch keinerlei Duftstoffe erhielten. Der Placebo-Inhalationsstift wurde dreimal mit einem Interstimulusintervall von 3 min verabreicht.

### b) Gruppe "Progressive Muskelrelaxation (PMR) nach Jacobson" (Vergleich)

Während der Intervention wurden die Probanden angeleitet, schrittweise bestimmte Körpermuskeln sequentiell anzuspannen und wieder loszulassen. Das Schema erfolgte dabei "von unten nach oben". Dabei wurden Oberschenkel, Gesäß, Rücken, Arme und Schultern abwechselnd angespannt, danach wurde die eigentliche Relaxation durchgeführt, d. h. es wurden alle vorgenannten Muskelpartien gleichzeitig für jeweils 10 Sekunden angespannt. Gegen Ende jeder Anspannung wurden die Probanden angehalten, die Spannung zu intensivieren. Insgesamt durchliefen die Probanden drei Anspannungszyklen mit einem jeweiligen zweiminütigen Interstimulusintervall.

### c) Gruppe Inhalationsstift mit Aromaölzusammensetzung (Erfindung)

Die Applikation des erfindungsgemäßen Inhalationsstifts mit der Zusammensetzung erfolgte liegend in entspannter Körperposition. Die Probanden platzierten den Inhalationsstift unter einem Nasenloch und hielten das andere Nasenloch mit einem Finger zu. Dabei atmeten die Probanden einmal tief ein, wobei die Zusammensetzung bzw. die darin enthaltenen Duftstoffe für kurze Zeit in der Nase gehalten und anschließend durch den Mund wieder ausgeatmet wurden. Der Inhalationsstift wurde dreimal mit einem Interstimulusintervall von 3 min verabreicht. Nach Abschluss der Intervention wurde zu den Zeitpunkten t₃₆₋₄₁ erneut eine Messung von Blutdruck und Herzrate vorgenommen. Zum Zeitpunkt t₄₂ wurde erneut die subjektiv empfundene Befindlichkeit der Probanden bestimmt, indem der Fragebogen ein zweites Mal ausgefüllt wurde. Nach 45 min zum Zeitpunkt t₄₅ war die Studie abgeschlossen.

### Ergebnisse

Nachfolgend sind die ermittelten Studienergebnisse in Bezug auf die Befindlichkeit, die Herzrate sowie den Blutdruck vor und nach der jeweiligen Intervention von allen Probandengruppen aufgeführt.

Angegeben sind jeweils die in den einzelnen Gruppen erhaltenen Mittelwerte sowie die diesbezügliche Standardabweichung. Der nachfolgenden Tabelle 2 sind die Ergebnisse der Messung des systolischen Blutdrucks, Tabelle 3 die Ergebnisse der Messung des diastolischen Blutdrucks, Tabelle 4 die Messwerte der Herzrate sowie Tabelle 5 die Messwerte für die subjektive Beurteilung der Befindlichkeit zu entnehmen.

**Tabelle 2: Mittelwerte und Standardabweichungen des systolischen Blutdrucks**

| **Bedingung** | **Mittelwert vorher [mm Hg] (Standardabweichung)** | **Mittelwert nachher [mm Hg] (Standardabweichung)** | **Differenz [mm Hg] (vorher/ nachher)** |
|---|---|---|---|
| Kontrollgruppe | 119,13 (17,89) | 117,38 (17,08) | -1,75 |
| Progressive Muskelrelaxation | 123,47 (18,26) | 116,33 (17,8) | -7,14 |
| Inhalationsstift | 126,04 (15,76) | 114,18 (12,67) | -11,86 |

Was den systolischen Blutdruck anbelangt, war der Effekt bei der Probandengruppe, welche den Inhalationsstift erhalten hatte, am größten. Der systolische Blutdruck war nach der Intervention um durchschnittlich 11,86 mm Hg gesunken. Bei der Kontrollgruppe hingegen war keine nennenswerte Senkung des Blutdrucks zu verzeichnen. Auch bei den Probanden, welche während der Intervention die wissenschaftliche Entspannungstechnik der Progressiven Muskelrelaxation nach Jacobson angewendet hatten, trat zwar eine stärkere Blutdrucksenkung im Vergleich zur Kontrollgruppe ein, im Vergleich zu der Probandengruppe, welche den wirkstoffhaltigen Inhalationsstift appliziert hatte, war der Effekt jedoch signifikant geringer.

**Tabelle 3: Mittelwerte und Standardabweichungen des diastolischen Blutdrucks**

| **Bedingung** | **Mittelwert vorher [mm Hg] (Standardabweichung)** | **Mittelwert nachher [mm Hg] (Standardabweichung)** | **Differenz (vorher/ nachher)** |
|---|---|---|---|
| Kontrollgruppe | 74,44 (12,49) | 73,58 (13,03) | -0,86 |
| Progressive Muskelrelaxation | 79,83 (12,94) | 73,56 (12,03) | -6,24 |
| Inhalationsstift | 80,09 (13,06) | 72,09 (10,33) | -8 |

Auch in Bezug auf die Senkung des diastolischen Blutdrucks war der Effekt bei der Probandengruppe, welche den Inhalationsstift erhalten hatte, am größten. In der Kontrollgruppe konnte der diastolische Blutdruck hingegen kaum gesenkt werden. Bei der Probandengruppe, welche Progressive Muskelrelaxation praktiziert hatte, war eine mittlere Senkung des diastolischen Blutdrucks zu verzeichnen.

**Tabelle 4: Mittelwerte und Standardabweichungen der Herzrate**

| **Bedingung** | **Mittelwert vorher [mm Hg] (Standardabweichung)** | **Mittelwert nachher [mm Hg] (Standardabweichung)** | **Differenz (vorher/ nachher)** |
|---|---|---|---|
| Kontrollgruppe | 71,36 (11,73) | 68,64 (11,31) | -2,72 |
| Progressive Muskelrelaxation | 71,18 (12,72) | 66,13 (8,69) | -5,64 |
| Inhalationsstift | 68,38 (12,47) | 62,27 (10,92) | -6,11 |

Auch bezüglich der Herzrate konnten deutliche Unterschiede von Kontrollgruppe zu der Probandengruppe, welche den Inhalationsstift verabreicht bekommen hatte, und den Probanden, welche Progressive Muskelentspannung praktiziert hatten, verzeichnet werden. Der größte Effekt war erneut bei der Probandengruppe mit dem Inhalationsstift aufgetreten. Bei der Kontrollgruppe hingegen wurde der geringste Rückgang der Herzrate verzeichnet.

**Tabelle 5: Mittelwerte und Standardabweichungen der Befindlichkeit**

| **Bedingung** | **Mittelwert vorher (Standardabweichung)** | **Mittelwert nachher (Standardabweichung)** | **Differenz (vorher/ nachher)** |
|---|---|---|---|
| Kontrollgruppe | 54,2 (9,2) | 57,67 (8,09) | 3,47 |
| Progressive Muskelrelaxation | 57,27 (7,19) | 61,27 (6,61) | 4 |
| Inhalationsstift | 55,33 (8,59) | 66,07 (7,55) | 10,74 |

Überraschenderweise hat sich auch bezüglich der Befindlichkeit gezeigt, dass bei den Probandengruppen, welche den Inhalationsstift appliziert hatten, bereits nach einem kurzen Interventionsintervall von 10 Minuten bzw. nach dreimaliger Applikation des Inhalationsstifts die Befindlichkeit bzw. das Wohlbefinden deutlich gesteigert werden konnte. Besonders überraschend war in diesem Zusammenhang, dass auch das wissenschaftlich anerkannte Entspannungsverfahren der Progressiven Muskelrelaxation keine nennenswerte Steigerung des Wohlbefindens bewirkte, sondern das Ergebnis vergleichbar mit der Kontrollgruppe war. Dass mit dem erfindungsgemäßen Inhalationsstift eine Steigerung des Wohlbefindens erzielt werden kann, welche sogar wissenschaftlich anerkannte Entspannungstechniken übertrifft, war so nicht erwartet worden.

Insgesamt ergeben sich folglich bei der Analyse der vorgenannten Messwerte deutliche Unterschiede vor und nach der experimentellen Intervention zwischen den einzelnen Gruppen. Sowohl in Bezug auf physische Faktoren, d. h. eine Senkung von Blutdruck und Herzrate, als auch in Bezug auf psychologische Faktoren, d. h. die Steigerung des Wohlbefindens, lassen sich mit einem erfindungsgemäßen Inhalationsstift, welcher eine Zusammensetzung auf Basis von Lavendelöl, Vetiveröl und Mandarinenöl enthält, die besten Ergebnisse erzielen.

Inhalationsstifte der vorgenannten Art eignen sich somit in hervorragender Weise, die mit chronischem Stress einhergehende Symptomatik von kardiovaskulären Erkrankungen, wie Bluthochdruck und Herzrasen, sowie psychosomatischen Symptomen, wie Abgeschlagenheit, Antriebslosigkeit und Müdigkeit, auf eine nebenwirkungsarme, vorzugsweise nebenwirkungsfreie, und darüber hinaus in der Handhabung äußerst komfortable Art und Weise zu behandeln.

### 2. Inhalationsstift mit einer Aromaölkombination von Bergamotteöl, Zitronenöl, Cedernholzöl, Muskatellersalbeiöl und Vetiveröl

Der erfindungsgemäße Inhalationsstift enthielt zu Testzwecken 0,8 ml einer Zusammensetzung, welche 34 Gew.-% Bergamotteöl, 28 Gew.-% Zitronenöl, 14 Gew.-% Cedernholzöl, 14 Gew.-% Muskatellersalbeiöl sowie 5 Gew.-% Vetiveröl, jeweils bezogen auf die Wirkstoffzusammensetzung, aufwies. Im Rahmen der von der Anmelderin durchgeführten Anwendungsstudien wurde der Einfluss dieser Kombination von ätherischen Ölen auf das Herz/KreislaufSystem von Personen, welche unter chronischem Stress litten, untersucht. Als Vergleich diente ein erfindungsgemäßer Inhalationsstift ohne ätherische Öle ("Placebostift"). Darüber hinaus wurde zu Vergleichszwecken ermittelt, wie stark der Einfluss der wissenschaftlich anerkannten Entspannungstechnik der Progressiven Muskelrelaxation nach Jacobson auf das Herz/KreislaufSystem ist.

Dazu wurde eine Probandengruppe von 45 Personen, von denen 33 weiblich und 12 männlich waren, mit einem Alter im Bereich von 18 bis 65 Jahren herangezogen. Die Probanden wurden unter der Voraussetzung zu der Studie zugelassen, dass bei ihnen das Kriterium eines erhöhten chronischen Stresslevels erfüllt ist. Um die erhaltenen Ergebnisse nicht zu verfälschen, wurden medizinisch-klinische Diagnosen, wie Blutdruckerkrankungen, kardiovaskuläre Erkrankungen, Diabetes, Drogen- oder Medikamentenabhängigkeit, epileptische Anfälle, Schwangerschaft, psychische Erkrankungen und akute Erkältungen, bei den Probanden ausgeschlossen.

Das Studiendesign basierte auf einer placebokontrollierten, randomisierten, dreiarmigen Explorationsstudie mit Messwiederholung. Die Probanden wurden auf die nachfolgend in Tabelle 6 aufgeführten Vergleichsgruppen von jeweils 15 Probanden aufgeteilt:

**Tabelle 6: Experimentalbedingungen, Instruktionen und getestete Wirkungen**

| **Gruppe** | **Instruktion** | **Getestete Wirkung** |
|---|---|---|
| Kontroll-/Placebogruppe | Inhalationsstift ohne Wirkstoff | Ausschluss unspezifischer Effekte (Gewöhnung, Entspannung) |
| Progressive Muskelrelaxation (PMR) nach Jacobson | Wissenschaftlich anerkanntes Beruhigungs- und Entspannungsverfahren | Vergleich der erzielten physiologischen und psychologischen Entspannung bei der Indikation von chronischem Stress |
| Inhalationsstift | Aromatherapie auf Basis der Wirkstoffkombination Bergamotteöl, Zitronenöl, Cedernholzöl, Muskattellersalbeiöl und Vetiveröl | Untersuchung der Duftstoffwirkung in Bezug auf die physiologische und psychologische Entspannung bei der Indikation von chronischem Stress |

### Studienablauf

Die Probanden wurden jeweils einzeln getestet, wobei die Studiendauer insgesamt 53 min betrug. Die Studie begann mit dem Empfang der Probanden zum Zeitpunkt t₀.

Nach 12 min zum Zeitpunkt t₁₂ beurteilten die Probanden ihre aktuelle Befindlichkeit bzw. ihr Wohlbefinden anhand eines auszufüllenden Fragebogens. Der Fragebogen enthielt 20 die Befindlichkeit bzw. das Wohlbefinden betreffende Adjektive, umfassend sowohl positive als auch negative Affekte, wobei die Probanden auf einer vierstufigen Skala beurteilten, ob die jeweiligen Adjektive "gar nicht", "kaum", "etwas" oder "sehr" zutrafen. Im Rahmen der Beurteilung konnten Werte im Bereich von 20 bis 80 erzielt werden, wobei "80" dem positivsten Wohlbefinden und "20" dem negativsten Wohlbefinden entsprach.

Anschließend wurden nach 15 bis 20 min der Blutdruck sowie die Herzrate mit Hilfe von automatischen oszillometrischen Oberarmmessgeräten (Fa. Hartmann, Heidenheim, Deutschland) bestimmt. Darüber hinaus wurde eine Speichelprobe zur Bestimmung des Kortisolspiegels genommen. Bei Kortisol handelt es sich um ein Stress-assoziiertes Hormon, welches vom menschlichen Körper in Belastungssituationen ausgeschüttet wird.

25 min nach Beginn der Studie wurde zum Zeitpunkt t₂₅₋₃₅ die 10-minütige Intervention durchgeführt. Wie nachfolgend geschildert, unterschied sich die Intervention je nach Probandengruppe:

### a) Kontrollgruppe (Vergleich)

Die Intervention in der Kontrollgruppe bestand darin, dass die Probanden Placebo-Inhalationsstifte erhielten, welche bezüglich ihrer Form und Größe mit den erfindungsgemäßen Inhalationsstiften identisch waren, jedoch keinerlei Duftstoffe erhielten. Der Placebo-Inhalationsstift wurde dreimal mit einem Interstimulusintervall von 3 min verabreicht.

### b) Gruppe "Progressive Muskelrelaxation (PMR) nach Jacobson" (Vergleich)

Während der Intervention wurden die Probanden angeleitet, schrittweise bestimmte Körpermuskeln sequentiell anzuspannen und wieder loszulassen. Das Schema erfolgte dabei "von unten nach oben". Dabei wurden Oberschenkel, Gesäß, Rücken, Arme und Schultern abwechselnd angespannt, danach wurde die eigentliche Relaxation durchgeführt, d. h. es wurden alle vorgenannten Muskelpartien gleichzeitig für jeweils 10 Sekunden angespannt. Gegen Ende jeder Anspannung wurden die Probanden angehalten, die Spannung zu intensivieren. Insgesamt durchliefen die Probanden drei Anspannungszyklen mit einem jeweiligen zweiminütigen Interstimulusintervall.

### c) Gruppe Inhalationsstift mit Aromaölzusammensetzung (Erfindung)

Die Applikation des erfindungsgemäßen Inhalationsstifts mit der Zusammensetzung erfolgte liegend in entspannter Körperposition. Die Probanden platzierten den Inhalationsstift unter einem Nasenloch und hielten das andere Nasenloch mit einem Finger zu. Dabei atmeten die Probanden einmal tief ein, wobei die Zusammensetzung bzw. die darin enthaltenen Duftstoffe für kurze Zeit in der Nase gehalten und anschließend durch den Mund wieder ausgeatmet wurden. Der Inhalationsstift wurde dreimal mit einem Interstimulusintervall von 3 min verabreicht.

Nach Abschluss der Intervention wurde zu den Zeitpunkten t₃₉₋₄₄ erneut eine Messung von Blutdruck und Herzrate vorgenommen. Zum Zeitpunkt t₄₅₋₄₈ wurde erneut die subjektiv empfundene Befindlichkeit der Probanden bestimmt, indem der Fragebogen ein zweites Mal ausgefüllt wurde. Schließlich gaben die Probanden zum Zeitpunkt t₄₉₋₅₂ eine zweite Speichelprobe ab. Nach 53 min zum Zeitpunkt t₅₃ war die Studie abgeschlossen.

### Ergebnisse

Nachfolgend sind die ermittelten Studienergebnisse in Bezug auf die Befindlichkeit, die Herzrate, den Kortisollevel sowie den Blutdruck vor und nach der jeweiligen Intervention von allen Probandengruppen aufgeführt.

Angegeben sind jeweils die in den einzelnen Gruppen erhaltenen Mittelwerte sowie die diesbezügliche Standardabweichung. Der nachfolgenden Tabelle 7 sind die Ergebnisse der Messung des systolischen Blutdrucks, Tabelle 8 die Ergebnisse der Messung des diastolischen Blutdrucks, Tabelle 9 die Messwerte der Herzrate sowie Tabelle 10 die Messwerte bezüglich der Bestimmung des Kortisolspiegels zu entnehmen. Tabelle 11 enthält schließlich die erhaltenen Messwerte für die subjektive Beurteilung der Befindlichkeit.

**Tabelle 7: Mittelwerte und Standardabweichungen des systolischen Blutdrucks**

| **Bedingung** | **Mittelwert vorher [mm Hg] (Standardabweichung)** | **Mittelwert nachher [mm Hg] (Standardabweichung)** | **Differenz [mm Hg] (vorher/ nachher)** |
|---|---|---|---|
| Kontrollgruppe | 117,07 (12,2) | 117,29 (13,27) | 0,22 |
| Progressive Muskelrelaxation | 117,64 (11,73) | 114,51 (12,98) | -3,13 |
| Inhalationsstift | 122,98 (18,85) | 108,49 (17,94) | -14,49 |

Was den systolischen Blutdruck anbelangt, war der Effekt bei der Probandengruppe, welche den erfindungsgemäßen Inhalationsstift erhalten hatte, am größten. Der systolische Blutdruck war nach der Intervention um durchschnittlich 14,49 mm Hg gesunken. Bei der Kontrollgruppe hingegen war keine nennenswerte Senkung des Blutdrucks zu verzeichnen. Auch bei den Probanden, welche während der Intervention die wissenschaftliche Entspannungstechnik der Progressiven Muskelrelaxation nach Jacobson angewendet hatten, trat im Vergleich zu der Probandengruppe, welche den Inhalationsstift appliziert hatte, nur eine geringe Senkung des systolischen Blutdrucks ein.

**Tabelle 8: Mittelwerte und Standardabweichungen des diastolischen Blutdrucks**

| **Bedingung** | **Mittelwert vorher [mm Hg] (Standardabweichung)** | **Mittelwert nachher [mm Hg] (Standardabweichung)** | **Differenz (vorher/ nachher)** |
|---|---|---|---|
| Kontrollgruppe | 74,22 (8,55) | 73,58 (9,19) | -0,64 |
| Progressive Muskelrelaxation | 71,96 (8,14) | 67,38 (7,09) | -4,58 |
| Inhalationsstift | 74,82 (10,4) | 68,36 (10,69) | -6,47 |

Auch in Bezug auf die Senkung des diastolischen Blutdrucks war der Effekt bei der Probandengruppe, welche den erfindungsgemäßen Inhalationsstift erhalten hatte, am größten. In der Kontrollgruppe konnte der diastolische Blutdruck hingegen kaum gesenkt werden. Bei der Probandengruppe, welche Progressive Muskelrelaxation praktiziert hatte, wurde eine mittlere Senkung des diastolischen Blutdrucks beobachtet.

**Tabelle 9: Mittelwerte und Standardabweichungen der Herzrate**

| **Bedingung** | **Mittelwert vorher [mm Hg] (Standardabweichung)** | **Mittelwert nachher [mm Hg] (Standardabweichung)** | **Differenz (vorher/ nachher)** |
|---|---|---|---|
| Kontrollgruppe | 73,56 (11,61) | 72 (11,01) | -1,56 |
| Progressive Muskelrelaxation | 74,67 (15,93) | 71,82 (14,86) | -2,85 |
| Inhalationsstift | 72,09 (11,19) | 64,64 (9,29) | -7,45 |

Auch bezüglich der Herzrate konnten deutliche Unterschiede von Kontrollgruppe zu der Probandengruppe, welche den erfindungsgemäßen Inhalationsstift verabreicht bekommen hatte, sowie den Probanden, welche Progressive Muskelentspannung praktiziert hatten, verzeichnet werden. Der größte Effekt war erneut bei der Probandengruppe mit dem erfindungsgemäßen Inhalationsstift aufgetreten. Bei der Kontrollgruppe hingegen wurde die geringste Senkung der Herzrate verzeichnet.

**Tabelle 10: Mittelwerte und Standardabweichungen des Kortisollevels**

| **Bedingung** | **Mittelwert vorher (Standardabweichung)** | **Mittelwert nachher (Standardabweichung)** | **Differenz (vorher/ nachher)** |
|---|---|---|---|
| Kontrollgruppe | 0,29 (0,12) | 0,29 (0,13) | 0 |
| Progressive Muskelrelaxation | 0,29 (0,13) | 0,27 (0,14) | -0,02 |
| Inhalationsstift | 0,3 (0,17) | 0,25 (0,14) | -0,05 |

Auch die Untersuchung des Kortisollevels hat ergeben, dass die Anwendung des erfindungsgemäßen Inhalationsstifts einen positiven Effekt auf die Linderung Stress-assoziierter Symptome besitzt. Bei der Probandengruppe, welche den erfindungsgemäßen Inhalationsstift mit der Aromaölzusammensetzung angewendet hatte, konnte der stärkste Rückgang des Kortisolgehalts im Speichel verzeichnet werden. Bei der Kontrollgruppe hingegen wurde gar kein Rückgang des Kortisollevels verzeichnet. Bei der Probandengruppe, welche die Progressive Muskelrelaxation angewendet hatte, war der Rückgang des Kortisolgehalts ebenfalls deutlich geringer als bei der Probandengruppe, die den erfindungsgemäßen Inhalationsstift appliziert hatte.

**Tabelle 11: Mittelwerte und Standardabweichungen der Befindlichkeit**

| **Bedingung** | **Mittelwert vorher (Standardabweichung)** | **Mittelwert nachher (Standardabweichung)** | **Differenz (vorher/ nachher)** |
|---|---|---|---|
| Kontrollgruppe | 51,4 (8,16) | 55,13 (8,13) | 3,73 |
| Progressive Muskelrelaxation | 53,87 (11,21) | 57,2 (7,6) | 3,33 |
| Inhalationsstift | 55,13 (8,05) | 61,73 (5,28) | 6,6 |

Überraschenderweise hat sich auch bezüglich der Befindlichkeit bzw. des Wohlbefindens gezeigt, dass bei den Probanden, welche den Inhalationsstift appliziert hatten, bereits nach einem kurzen Interventionsintervall von 10 Minuten bzw. nach dreimaliger Applikation des Inhalationsstifts die Befindlichkeit bzw. das Wohlbefinden deutlich gesteigert werden konnte. Besonders überraschend war in diesem Zusammenhang, dass auch das wissenschaftlich anerkannte Entspannungsverfahren der Progressiven Muskelrelaxation keine nennenswerte Steigerung des Wohlbefindens bewirkte, sondern vergleichbar mit dem Ergebnis der Kontrollgruppe war.

Insgesamt ergeben sich folglich bei der Analyse der vorgenannten Messwerte deutliche Unterschiede vor und nach der experimentellen Intervention zwischen den einzelnen Gruppen. Sowohl in Bezug auf physische Faktoren, d. h. eine Senkung von Blutdruck, Herzrate und Kortisollevel, als auch in Bezug auf psychologische bzw. psychische Faktoren, d. h. die Steigerung des Wohlbefindens, lassen sich mit einem erfindungsgemäßen Inhalationsstift, welcher eine Zusammensetzung auf Basis von Bergamotteöl, Zitronenöl, Cedernholzöl, Muskatellersalbeiöl und Vetiveröl enthält, die besten Ergebnisse erzielen. Dies war in dieser ausgeprägten Form nicht vorhersehbar gewesen.

Auch Inhalationsstifte mit einer Zusammensetzung auf Basis von Bergamotteöl, Zitronenöl, Cedernholzöl, Muskatellersalbeiöl und Vetiveröl eignen sich somit in hervorragender Weise, die mit chronischem Stress einhergehende Symptomatik von kardiovaskulären Erkrankungen, wie Bluthochdruck und Herzrasen, sowie psychosomatischen Symptomen, wie Abgeschlagenheit, Antriebslosigkeit und Müdigkeit, auf eine nebenwirkungsarme, insbesondere nebenwirkungsfreie, und darüber hinaus in der Handhabung äußerst komfortable Art und Weise zu behandeln.

### 3. Inhalationsstift mit einer Aromaölkombination von Pfefferminzöl und Grapefruitöl

Der erfindungsgemäße Inhaltionsstift enthielt zu Testzwecken 0,8 ml einer Zusammensetzung, welche 50 Gew.-% Pfefferminzöl und 50 Gew.-% Grapefruitöl, jeweils bezogen auf die Zusammensetzung, enthielt. Im Rahmen der von der Anmelderin durchgeführten Anwendungsstudien wurde der Einfluss dieser erfindungsgemäßen Kombination von ätherischen Ölen auf eine Gewichtsreduktion untersucht. Als Vergleich diente ein erfindungsgemäßer Inhalationsstift, welcher lediglich Lavendelöl enthielt ("Placebostift").

Dazu wurde eine Probandengruppe von 40 Personen, von denen 28 weiblich und 12 männlich waren, mit einem Alter im Bereich von 24 bis 61 Jahren herangezogen. Die Probanden wurden unter der Voraussetzung zu der Studie zugelassen, dass ihr BMI (*Body Mass Index)* zwischen 20 und 35 lag. Darüber hinaus wurden medizinisch-klinische Diagnosen, wie Blutdruckerkrankungen, kardiovaskuläre Erkrankungen, Diabetes, Drogen- oder Medikamentenabhängigkeit, epileptische Anfälle, Schwangerschaft oder psychische Erkrankungen, bei den Probanden ausgeschlossen.

### Studiendesign:

Das Studiendesign basierte auf einer placebokontrollierten, randomisierten prospektiven Dokumentationsstudie. Die Probanden wurden auf die nachfolgend in Tabelle 12 aufgeführten Vergleichsgruppen von jeweils 20 Probanden aufgeteilt:

**Tabelle 12: Experimentalbedingungen, Instruktionen und getestete Wirkungen**

| **Gruppe** | **Instruktion** | **Getestete Wirkung** |
|---|---|---|
| Kontroll-/Placebogruppe | Inhalationsstift mit Lavendelöl | Unspezifische Effekte (Motivation, Bewusstmachung) |
| Inhalationsstift | Aromatherapie auf Basis der Wirkstoffkombination Pfefferminzöl und Grapefruitöl zur Unterstützung der Gewichtsreduktion | Untersuchung der Duftstoffwirkung in Bezug auf die Unterstützung der Gewichtsreduktion (Fettverbrennung und Appetitreduktion) |

Die Studie erstreckte sich insgesamt über einen Zeitraum von 30 Tagen, wobei die Probanden mehrmals täglich entweder den erfindungsgemäßen Inhalationsstift oder den Placebostift anwendeten. Die Applikationsweise der Inhalationsstifte war dabei wie folgt: Alle Probanden applizierten den jeweils zu testenden Inhalationsstift 3 bis 5 Mal etwa 5 bis 10 Minuten vor jeder Nahrungsaufnahme. Darüber hinaus erfolgte eine 3- bis 5-malige Inhalation zusätzlich alle 2 Stunden. Um die Motivation bzw. Spannung der Probanden nicht zu beeinflussen, sollten sie sich während der Studie nicht zu Hause wiegen. Darüber hinaus unternahmen die Probanden keine Änderungen in Bezug auf ihren übrigen Lebensrhythmus bzw. Lebensstil, um die Wirkung der Inhalationsstifte nicht zu verfälschen. Insbesondere erhöhten die Probanden weder ihr Sportpensum noch schränkten sie ihre Kalorienzufuhr ein bzw. änderten ihr Essverhalten.

Um den Einfluss der Inhalationsstifte auf den Gewichtsverlust zu analysieren, wurden die Probanden vor Beginn der Studie sowie nach 30 Tagen gewogen. Darüber hinaus nahmen die Probanden an einer Endbefragung teil, im Rahmen derer sie in Bezug auf die wahrgenommene subjektive Wirkung der Inhalationsstifte befragt wurden.

### Ergebnisse:

Nachfolgend sind die ermittelten Studienergebnisse in Bezug auf das Gewicht vor und nach der Studie von allen Probanden aufgeführt. Darüber hinaus sind nachfolgend die Ergebnisse in Bezug auf die Endbefragung im Detail erläutert.

Der nachfolgenden Tabelle 13 sind die Ergebnisse der Messung des Gewichts der Probanden am Anfang und am Ende der Studie zu entnehmen. Angegeben sind jeweils die in den einzelnen Gruppen erhaltenen Mittelwerte sowie die diesbezügliche Standardabweichung.

**Tabelle 13: Mittelwerte und Standardabweichungen des Gewichtverlusts**

| **Bedingung** | **Durchschnittlicher Gewichtsverlust [kg] (Standardabweichung)** |
|---|---|
| Kontrollgruppe | 1,3 (0,5) |
| Inhalationsstift | 2,4 (0,3) |

Wie die vorstehenden Ausführungen zeigen, konnte durch die bloße Anwendung des erfindungsgemäßen Inhalationsstifts ohne begleitende Maßnahmen zur Gewichtsreduktion, wie beispielsweise einer Ernährungsumstellung oder mehr Sport, eine signifikant größere Gewichtsreduktion erzielt werden, als es für den Placebostift, welcher lediglich Lavendelöl enthielt, der Fall war.

Darüber hinaus hat sich im Rahmen der Endbefragung gezeigt, dass die Probanden, welche den erfindungsgemäßen Inhalationsstift angewendet hatten, während des Zeitraums der Studie weniger Hunger verspürt hatten und insgesamt zu einer geringeren Nahrungsaufnahme tendiert hatten. Bei der Kontrollgruppe, welche im Mittel deutlich weniger abgenommen hatte als die Probandengruppe mit dem erfindungsgemäßen Inhalationsstift, gaben deutlich weniger Probanden an, während des Versuchszeitraums weniger Hunger gehabt zu haben bzw. zu einer geringeren Nahrungsaufnahme tendiert zu haben.

Insgesamt hat sich im Rahmen der Studien gezeigt, dass die Anwendung eines erfindungsgemäßen Inhalationsstiftes mit der vorgenannten Aromaölkombination auf Basis von Pfefferminzöl und Grapefruitöl bereits ohne zusätzliche unterstützende Maßnahmen bezüglich der Ernährung und Sport zu einer Gewichtsreduktion führt. Somit können erfindungsgemäße Inhalationsstifte bereits allein zur Gewichtsreduktion eingesetzt werden, darüber hinaus aber auch eine sinnvolle Ergänzung zu Diäten und Ernährungsumstellungen darstellen.

### 4. Inhalationsstift mit einer Aromaölkombination von Pfefferminzöl, Rosmarinöl und Lavendelöl

Der erfindungsgemäße Inhalationsstift enthielt zu Testzwecken 0,8 ml einer Zusammensetzung, welche 60 Gew.-% Pfefferminzöl, 30 Gew.-% Rosmarinöl sowie 10 Gew.-% Lavendelöl, jeweils bezogen auf die Zusammensetzung, enthielt. Im Rahmen der von der Anmelderin durchgeführten Anwendungsstudien wurde der Einfluss der vorgenannten erfindungsgemäßen Kombination von ätherischen Ölen auf menstruelle Schmerzen und Beschwerden von Frauen untersucht.

Dazu wurde eine Probandengruppe von 30 Frauen mit einem Alter von 18 bis 50 Jahren herangezogen. Die Probandinnen wurden unter der Voraussetzung zu der Studie zugelassen, dass sie regelmäßige Menses hatten und unter mindestens starken Menstruationsbeschwerden leiden. Sie durften darüber hinaus keine rezeptpflichtige Medikation zur Milderung der monatlichen Schmerzen einnehmen. Rezeptfreie Medikationen, wie handelsübliche Analgetika, waren hingegen erlaubt.

### Studienablauf:

Das Studiendesign sah vor, dass die Probandinnen ihre Schmerzen bzw. Beschwerden über zwei Menstruationszyklen beobachteten, jedoch nur während eines Zyklus den erfindungsgemäßen Inhalationsstift anwendeten. Darüber hinaus konnten die Probandinnen nach Bedarf während beider Zyklen ihre üblichen Maßnahmen zur Beschwerdelinderung ergreifen. Der erfindungsgemäße Inhalationsstift konnte damit sozusagen auch als Add-on zur Schmerz- und Beschwerdemilderung eingesetzt werden.

Die Anwendung des Inhalationsstifts erfolgte während der Akutphase der Beschwerden bzw. Schmerzen alle 10 Minuten. Pro Anwendung wurde 10 Mal tief an dem Inhalationsstift inhaliert.

Die Probandinnen wurden dazu angehalten, die subjektiv empfundene Schmerzintensität während der Akutphasen der Beschwerden sowie die Befindlichkeit während beider Zyklen zu dokumentieren und beide Zyklen miteinander zu vergleichen.

### Ergebnisse:

In Bezug auf die Schmerzintensität hat sich gezeigt, dass alle Probandinnen durch die zusätzliche Anwendung des erfindungsgemäßen Inhalationsstifts die Schmerzen weiter lindern konnten. Insbesondere hat sich im Rahmen der Studien gezeigt, dass die Anwendung des Inhalationsstifts bewirkt, dass auch die übrigen ergriffenen Maßnahmen, wie die Einnahme handelsüblicher Analgetika oder Wärmetherapien, in ihrer Wirkeffizienz signifikant verbessert werden können. Insbesondere hat sich im Rahmen der Studie gezeigt, dass die Pharmakodynamik bzw. Pharmakokinetik handelsüblicher verbessert bzw. beschleunigt werden kann, d. h. die Latenz, also die Zeit bis zum Wirkeintritt des Analgetikum, und die Schmerzdauer bis zur Schmerzfreiheit signifikant verkürzt werden können. Auch die Befindlichkeit der Probandinnen, insbesondere das allgemeine Wohlbefinden, konnte durch den Einsatz des erfindungsgemäßen Inhalationsstifts deutlich verbessert werden. Unter Anwendung des erfindungsgemäßen Inhalationsstifts fühlten sich die Probandinnen während ihrer Menstruation deutlich entspannter und besser.

Insgesamt hat sich somit im Rahmen der Anwendungsstudien gezeigt, dass sich durch den Einsatz erfindungsgemäßer Inhalationsstifte die Behandlung von Schmerzen bzw. schmerzhaften Beschwerden deutlich verbessern lässt. Insbesondere eignet sich der erfindungsgemäße Inhalationsstift auch als unterstützende Maßnahme zur Wirkbeschleunigung handelsüblicher Analgetika zur Behandlung von Schmerzen bzw. Schmerzzuständen, wie sie z. B. im Rahmen von Menstruationsbeschwerden auftreten.

### 5. Studien zu weiteren Zusammensetzungen

Im Rahmen von weiteren Studien wurde der Einfluss weiterer Zusammensetzungen in Bezug auf die Behandlung von verschiedenen Erkrankungen untersucht. Die Inhalationsstifte wurden ebenfalls wie oben beschrieben mit den jeweiligen Zusammensetzungen beschickt. Probandengruppen, die unter den jeweils aufgeführten Erkrankungen bzw. Symptomen litten, applizierten die jeweilige Zusammensetzung ebenfalls wie oben beschrieben mit dem jeweiligen Inhalationsstift.

### a) Zusammensetzung auf Basis von Pfefferminzöl, Rosmarinöl und Lavendelöl zur Schmerztherapie

Die Zusammensetzungen, mit welchen die Inhalationsstifte ausgerüstet wurden, enthielten 60 Gew.-% Pfefferminzöl, 30 Gew.-% Rosmarinöl und 10 Gew.-% Lavendelöl. Durch Applikation des Inhalationsstifts mit der vorgenannten Zusammensetzung konnten die Schmerzen bzw. Schmerzzustände der Probanden signifikant gelindert werden.

### b) Zusammensetzung auf Basis von Grapefruitöl und Pfefferminzöl zur Gewichtsreduktion

Die Zusammensetzungen, mit welchen die Inhalationsstifte ausgerüstet wurden, enthielten 50 Gew.-% Grapefruitöl und 50 Gew.-% Pfefferminzöl. Durch Applikation des Inhalationsstifts mit der vorgenannten Zusammensetzung über einen Zeitraum von 4 Wochen bei gleichzeitig ausgewogener Ernährung ohne die Befolgung eines strikten Diätprogramms konnten die ausgewählten Probanden, deren BMI im Bereich von 23 bis 27 lag, ihr Gewicht durchschnittlich um 8 %, bezogen auf das Ausgangsgewicht, reduzieren. Die Applikation eines Inhalationsstifts mit einer Zusammensetzung auf Basis von Grapefruitöl und Pfefferminzöl stellt folglich eine wirksame unterstützende Maßnahme zur Gewichtsreduktion bzw. zur Behandlung von Übergewicht dar.

### c) Zusammensetzung auf Basis von Eukalyptusöl, Pfefferminzöl und Thymianöl zur Behandlung von Erkältungen

Die Zusammensetzungen, mit welchen die Inhalationsstifte ausgerüstet wurden, enthielten 80 Gew.-% Eukalyptusöl, 10 Gew.-% Pfefferminzöl und 10 Gew.-% Thymianöl. Probanden, welche unter Erkältungskrankheiten litten, applizierten den Inhalationsstift mehrmals täglich je nach persönlichem Bedarf. Durch Applikation des Inhalationsstifts konnten klassische Erkältungssymptome, wie geschwollene Nasenschleimhäute, eine erschwerte Nasenatmung sowie Entzündungen im Mund/Rachen-Raum, signifikant gelindert werden. Insgesamt verhalf der Inhalationsstift zu einem schnellen Rückgang der Erkältungssymptome und einer schnellen Regeneration.

### d) Zusammensetzung auf Basis von Zitronenöl, Pfefferminzöl, Rosmarinöl, Grapefruitöl, Pfefferöl und Basilikumöl

Die Zusammensetzungen, mit welchen die Inhalationsstifte ausgerüstet wurden, enthielten 25 Gew.-% Zitronenöl, 22 Gew.-% Pfefferminzöl, 22 Gew.-% Rosmarinöl, 19 Gew.-% Grapefruitöl, 7 Gew.-% Pfefferöl und 5 Gew.-% Basilikumöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Verbesserung der inneren Ausgeglichenheit bei.

### e) Zusammensetzung auf Basis von Pfefferminzöl, Vetiveröl, Wacholderbeeröl und Thymianöl

Die Zusammensetzungen, mit welchen die Inhalationsstifte ausgerüstet wurden, enthielten 35 Gew.-% Pfefferminzöl, 30 Gew.-% Vetiveröl, 30 Gew.-% Wacholderbeeröl und 5 Gew.-% Thymianöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Verbesserung der inneren Ausgeglichenheit bei.

### f) Zusammensetzung auf Basis von Pfefferminzöl, Cypressenöl, Geraniumöl und Gingergrasöl

Die Zusammensetzungen, mit welchen die Inhalationsstifte ausgerüstet wurden, enthielten 48 Gew.-% Pfefferminzöl, 34 Gew.-% Cypressenöl, 14 Gew.-% Geraniumöl und 4 Gew.-% Gingergrasöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Verbesserung der inneren Ausgeglichenheit bei.

### g) Zusammensetzung auf Basis von Pfefferminzöl, Rosmarinöl und Zimtrindenöl

Die Zusammensetzungen, mit welchen die Inhalationsstifte ausgerüstet wurden, enthielten 86 Gew.-% Pfefferminzöl, 11 Gew.-% Rosmarinöl und 3 Gew.-% Zimtrindenöl. Durch Applikation eines Inhalationsstifts mit der vorgenannten Zusammensetzung konnte insgesamt das Wohlbefinden der Probanden gesteigert werden. Darüber hinaus ließen sich die Symptome von Herz/Kreislauf-Erkrankungen sowie von chronischem Stress signifikant lindern.

### h) Zusammensetzung auf Basis von Pfefferminzöl, Grapefruitöl und Zimtrindenöl

Die Zusammensetzungen, mit welchen die Inhalationsstifte ausgerüstet wurden, enthielten 90 Gew.-% Pfefferminzöl, 8 Gew.-% Grapefruitöl und 4 Gew.-% Zimtrindenöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Steigerung von Konzentration und Leistungsbereitschaft bei.

### i) Zusammensetzung auf Basis von Bergamotteöl, Majoranöl, Lavendelöl und Orangenöl

Die Zusammensetzungen, mit welchen die Inhalationsstifte ausgerüstet wurden, enthielten 40 Gew.-% Bergamotteöl, 20 Gew.-% Majoranöl, 20 Gew.-% Lavendelöl und 20 Gew.-% Orangenöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Steigerung von Konzentration und Leistungsbereitschaft bei.

### j) Zusammensetzung auf Basis von Rosmarinöl, Pfefferminzöl und Basilikumöl

Die Zusammensetzungen, mit welchen die Inhalationsstifte ausgerüstet wurden, enthielten 49 Gew.-% Rosmarinöl, 45 Gew.-% Pfefferminzöl und 6 Gew.-% Orangenöl. Durch Applikation eines Inhalationsstifts mit der vorgenannten Zusammensetzung konnte insgesamt das Wohlbefinden der Probanden gesteigert werden. Insbesondere trug die Applikation der Zusammensetzungen zu einer Steigerung von Konzentration und Leistungsbereitschaft bei.

## Patentansprüche

1. Inhalationsstift (1) zur Inhalation durch die Nase mit einer langgestreckten Gehäusehülse (2) und einem stabförmigen Speichermittel (3) zum Speichern mindestens eines ätherischen Öls, wobei die Gehäusehülse (2) einen inneren Aufnahmeraum (4) zur Aufnahme des Speichermittels (3) aufweist,
wobei innenseitig in der Gehäusehülse (2) wenigstens ein in radialer Richtung in den Aufnahmeraum (4) ragender Fixiervorsprung zur reibschlüssigen Fixierung des Speichermittels (3) in der Gehäusehülse (2) vorgesehen ist,
**dadurch gekennzeichnet,**
**dass** zum Verschluss der Gehäusehülse (2) eine in den hinteren Hülsenbereich (10) einsetzbare Stopfenhülse (17) vorgesehen ist, und dass in der Stopfenhülse (17) mit endseitigen Auflaufanschlägen (21) versehene, als Rippen (20) ausgebildete Fixiervorsprünge zur endseitigen Fixierung des Speichermittels (3) vorgesehen sind.

2. Inhalationsstift nach Anspruch 1, **dadurch gekennzeichnet, dass** in der Gehäusehülse (2) eine Mehrzahl von Fixiervorsprüngen vorgesehen sind und/oder dass das Speichermittel (3) ausschließlich an den Fixiervorsprüngen anliegt, ohne an den übrigen Innenwandungsbereichen (8) des Aufnahmeraums (4) anzuliegen und/oder dass das Speichermittel (3) durch insbesondere über den Umfang gleich beabstandete, vorzugsweise als langgestreckte Rippen (7) ausgebildete Fixiervorsprünge zentriert im Aufnahmeraum (4) angeordnet ist.

3. Inhalationsstift nach Anspruch 1 oder 2, **dadurch gekennzeichnet, dass** die Gehäusehülse (2) einen vorderen, insbesondere als Inhalationsbereich ausgebildeten Hülsenbereich (9) und einen hinteren, insbesondere als Griffbereich ausgebildeten Hülsenbereich (10) aufweist und/oder dass am vorderen Ende (11) des vorderen Hülsenbereichs (9) wenigstens eine in den Aufnahmeraum (4) führende, insbesondere mittige Nasenöffnung (12) vorgesehen ist, und dass vorzugsweise am vorderen Hülsenbereich (9) wenigstens eine insbesondere radial in den Aufnahmeraum (4) führende Seitenöffnung (13) vorgesehen ist.

4. Inhalationsstift nach einem der vorangehenden Ansprüche, **dadurch gekennzeichnet, dass** die Rippen (7) im Bereich des vorderen Endes (11) des vorderen Hülsenbereichs (9) vorgesehen sind, und dass vorzugsweise die Rippen (7) jeweils einen Anschlag (14) zur endseitigen Anlage und Fixierung des Speichermittels (3) in axialer Richtung und eine endseitige Auflaufschräge (16) aufweisen.

5. Inhalationsstift nach einem der vorangehenden Ansprüche, **dadurch gekennzeichnet,**
**dass** die Stopfenhülse (17) mit der Gehäusehülse (2) verrastet ist und dass vorzugsweise außenseitig an der Stopfenhülse (17) ein insbesondere umlaufender Rastvorsprung (22) und innenseitig am hinteren Hülsenbereich (10) eine umlaufende Raststufe (23) vorgesehen sind, und/oder
**dass** eine Verschlusskappe (24) zum Aufsetzen auf den vorderen Hülsenbereich (9) zum Verschluss der Nasen- und Seitenöffnungen (12, 13) vorgesehen ist und dass vorzugsweise am Übergang vom vorderen Hülsenbereich (9) zum hinteren Hülsenbereich (10) eine Schraubverbindung zum Verschrauben der Verschlusskappe (24) mit der Gehäusehülse (2) vorgesehen ist.

6. Inhalationsstift nach einem der vorangehenden Ansprüche, **dadurch gekennzeichnet, dass** der Inhalationsstift (1) eine Zusammensetzung zur Verwendung bei der prophylaktischen und/oder therapeutischen Behandlung von Erkrankungen enthält, wobei der Inhalationsstift in seinem Inneren ein Speichermittel, vorzugsweise ein textiles Speichermittel, aufweist, wobei das Speichermittel mit der Zusammensetzung versehen und/oder beaufschlagt ist und
- wobei die Zusammensetzung mindestens zwei voneinander verschiedene ätherische Öle, insbesondere mindestens drei voneinander verschiedene ätherische Öle, aufweist, wobei die ätherischen Öle ausgewählt sind aus der Gruppe von
Bergamotteöl (Citrus aurantium var. bergamia),
Cedernholzöl (Cupressus funebris Endlicher),
Cypressenöl (Cupressus sempervirens),
Eukalyptusöl (Eucalyptus globulus),
Geraniumöl (Pelargonium graveolens),
Gingergrasöl (Cymbopogon martinii var. sofia),
Lavendelöl (Lavandula angustifolia),
Majoranöl (Origanum majorana),
Mandarinenöl (Citrus reticulata),
Muskatellersalbeiöl (Salvia sclarea),
Orangenöl (Citrus aurantium var. dulcis),
Pfefferminzöl (Mentha piperita),
Pfefferöl (Piper nigrum),
Rosmarinöl (Rosmarinus officinalis),
Thymianöl (Thymus serpyllum),
Vetiveröl (Vetiveria zizanoides),
Wacholderbeeröl (Juniperus communis L.),
Zimtrindenöl (Cinnamomum ceylanicum),
Zitronenöl (Citrus limon),
Grapefruitöl (Citrus decumana),
Basilikumöl (Ocimum basilicum),
Fichtenöl (Picea excelsa)
sowie deren Mischungen und Kombinationen.

7. Inhalationsstift nach Anspruch 6,
wobei das Speichermittel porös und/oder luftdurchlässig ausgebildet ist; und/oder wobei das Speichermittel ein textiles Material umfasst und/oder hieraus gebildet ist, insbesondere auf Basis von Baumwolle, Cellulose oder Cellulosederivaten, vorzugsweise Celluloseacetaten, Glycerintriacetaten sowie deren Mischungen und Kombinationen; und/oder
wobei das Speichermittel ein Wasseraufnahmevermögen von mindestens 3 g/g, insbesondere mindestens 4 g/g, vorzugsweise mindestens 5 g/g, aufweist; und/oder
wobei das Speichermittel zylinderförmig oder stabförmig ausgebildet ist, insbesondere mit Längen von 10 bis 100 mm und/oder mit Dicken (Durchmessern) von 1 bis 20 mm; und/oder
wobei das Speichermittel ein Gewicht von 0,1 bis 10,0 g, insbesondere 0,2 bis 7,5 g, vorzugsweise 0,3 bis 5 g, aufweist.

8. Inhalationsstift nach Anspruch 6 oder 7,
wobei der Inhalationsstift und/oder das Speichermittel die Zusammensetzung in wirksamen, insbesondere therapeutisch wirksamen Mengen enthält bzw. enthalten; und/oder wobei der Inhalationsstift und/oder das Speichermittel die Zusammensetzung in Mengen von 0,01 bis 10,0 ml, insbesondere 0,1 bis 5 ml, vorzugsweise 0,2 bis 1 ml, enthält bzw. enthalten; und/oder
wobei der Inhalationsstift und/oder das Speichermittel die Zusammensetzung in Mengen von 0,005 bis 15 g, insbesondere 0,05 bis 7,5 g, vorzugsweise 0,01 bis 1,5 g, besonders bevorzugt 0,1 bis 1 g, enthält bzw. enthalten.

9. Inhalationsstift nach einem der Ansprüche 6 bis 8,
wobei der Inhalationsstift und/oder das Speichermittel die Zusammensetzung in relativen Mengen von 0,1 bis 300 Gew.-%, insbesondere 0,2 bis 150 Gew.-%, vorzugsweise 0,5 bis 100 Gew.-%, besonders bevorzugt 1 bis 80 Gew.-%, bezogen auf das Speichermittel, enthält bzw. enthalten; und/oder
wobei das Speichermittel bei inhalativer Applikation, die Zusammensetzung in wirksamen, insbesondere therapeutisch wirksamen Mengen freizusetzen imstande ist.

10. Inhalationsstift nach einem der Ansprüche 6 bis 9,
wobei die Zusammensetzung bei 20 °C und Atmosphärendruck (101.325 Pascal) eine Dichte (relative Dichte) im Bereich von 0,5 bis 1,5 g/cm³, insbesondere im Bereich von 0,6 bis 1,2 g/cm³, vorzugsweise im Bereich von 0,7 bis 1,1 g/cm³, aufweist; und/oder wobei die Zusammensetzung bei 20 °C und Atmosphärendruck (101.325 Pascal) flüssig ist und/oder im flüssigen Aggregatzustand vorliegt; und/oder
wobei die Zusammensetzung bei 20 °C einen Brechungsindex im Bereich von 1,300 bis 1,700, insbesondere im Bereich von 1,350 bis 1,650, vorzugsweise im Bereich von 1,400 bis 1,600, aufweist.

11. Inhalationsstift nach einem der Ansprüche 6 bis 10,
wobei die Zusammensetzung einen Anteil an VOC (Volatile Organic Compounds) von weniger als 1 Gew.-%, insbesondere von weniger als 0,5 Gew.-%, vorzugsweise von 0 Gew.-%, bezogen auf die Zusammensetzung, aufweist; und/oder
wobei die Zusammensetzung mittels Pressung und/oder Wasserdampfdestillation, insbesondere aus den entsprechenden Ausgangsdrogen und/oder Pflanzenteilen, wie Fruchtschalen, Früchten, Holz, Blättern, Zweigen, Zweigspitzen, Blüten, Blütenständen, Gras, Kraut, Wurzeln, Rinden oder dergleichen, erhalten ist; und/oder
wobei die Zusammensetzung volatil und/oder flüchtig ist, insbesondere wobei die Zusammensetzung bei 20 °C einen Dampfdruck oberhalb von 20 mbar, insbesondere oberhalb von 24 mbar, vorzugsweise oberhalb von 28 mbar, bevorzugt oberhalb von 30 mbar, besonders bevorzugt oberhalb von 40 mbar, ganz besonders bevorzugt oberhalb von 60 mbar, noch mehr bevorzugt oberhalb von 100 mbar, aufweist.

12. Inhalationsstift nach einem der Ansprüche 6 bis 11,
wobei die Zusammensetzung mindestens eine der folgenden Kombinationen von ätherischen Ölen umfasst:
(i) Pfefferminzöl/Rosmarinöl/Lavendelöl, insbesondere in gewichtsbezogenen Verhältnissen von 40-80/10-50/1-20, vorzugsweise zur prophylaktischen und/oder therapeutischen Behandlung von Schmerzzuständen;
(ii) Grapefruitöl/Pfefferminzöl, insbesondere in gewichtsbezogenen Verhältnissen von 30-70/30-70, vorzugsweise zur prophylaktischen und/oder therapeutischen Behandlung von Stoffwechselerkrankungen, insbesondere Diabetes oder Übergewicht;
(iii) Eukalyptusöl/Pfefferminzöl/Thymianöl, insbesondere in gewichtsbezogenen Verhältnissen von 60-90/5-20/5-20, vorzugsweise zur prophylaktischen und/oder therapeutischen Behandlung von Infekten, insbesondere Erkältungen, Rhinitis und Bronchitis;
(iv) Lavendelöl/Vetiveröl/Mandarinenöl, insbesondere in gewichtsbezogenen Verhältnissen von 20-50/20-50/15-40, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(v) Zitronenöl/Pfefferminzöl/Rosmarinöl/Grapefruitöl/Pfefferöl/Basilikumöl, insbesondere in gewichtsbezogenen Verhältnissen von 15-30/15-30/15-30/10-25/2-10/1-8;
(vi) Bergamotteöl/Zitronenöl/Cedernholzöl/Muskatellersalbeiöl/Vetiveröl, insbesondere in gewichtsbezogenen Verhältnissen von 25-40/20-35/10-20/10-20/5-20, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(vii) Pfefferminzöl/Vetiveröl/Wacholderbeeröl/Thymianöl, insbesondere in gewichtsbezogenen Verhältnissen von 25-45/20-40/20-40/1-15;
(viii) Pfefferminzöl/Cypressenöl/Geraniumöl/Gingergrasöl, insbesondere in gewichtsbezogenen Verhältnissen von 40-60/25-45/10-30/1-10;
(ix) Pfefferminzöl/Rosmarinöl/Zimtrindenöl, insbesondere in gewichtsbezogenen Verhältnissen von 65-95/1-15/1-10, vorzugsweise zur prophylaktischen und/oder therapeutischen Behandlung von kardiovaskulären Erkrankungen (Erkrankungen des Herz/Kreislauf-Systems), insbesondere Bluthochdruck (Hypertonie), und/oder psychosomatischen Erkrankungen, insbesondere Stress, physisch-psychischen Überlastungen, Entzugssyndromen und Schlafstörungen;
(x) Pfefferminzöl/Grapefruitöl/Rosmarinöl, insbesondere in gewichtsbezogenen Verhältnissen von 75-95/1-12/1-5;
(xi) Bergamotteöl/Majoranöl/Lavendelöl/Orangenöl, insbesondere in gewichtsbezogenen Verhältnissen von 30-50/10-30/10-30/10-30;
(xii) Rosmarinöl/Pfefferminzöl/Basilikumöl, insbesondere in gewichtsbezogenen Verhältnissen von 40-60/35-55/1-15.

13. Verfahren zur Montage eines wie in einem der vorangehenden Ansprüche definierten Inhalationsstiftes (1), wobei das Speichermittel (3) im unbeladenen Zustand zunächst in den Aufnahmeraum (4) eingesetzt wird, wobei der Aufnahmeraum (4) anschließend geschlossen wird und wobei die Flüssigkeit nach dem Schließen des Aufnahmeraums über die Nasenöffnung (12) und/oder die Seitenöffnung (13) in das Speichermittel (3) eingebracht wird.

14. Verfahren nach Anspruch 13, wobei zumindest während des Einbringens der Flüssigkeit in das Speichermittel (3) ein Unterdruck, insbesondere zumindest in der Gehäusehülse (2), angelegt wird und/oder vorgesehen ist.

15. Verfahren nach Anspruch 13 oder 14, wobei das Speichermittel (3) im unbeladenen Zustand zunächst in den Aufnahmeraum (4) eingesetzt wird, wobei der Aufnahmeraum (4) anschließend durch Einsetzen der Stopfenhülse (17) in den hinteren Hülsenbereich (10) geschlossen wird und wobei das Speichermittel (3) in seiner Endmontagelage aufgrund der eingeschobenen Stopfenhülse (17) an beiden Enden in Längsrichtung fixiert wird.

## Claims

1. An inhalation stick (1) for inhalation through the nose, having an elongate housing sleeve (2) and a rodshaped storage means (3) for storing at least one essential oil, wherein the housing sleeve (2) comprises an inner compartment (4) to accommodate the storage means (3),
wherein at least one fixing projection which protrudes into the compartment (4) in the radial direction is provided in the interior in the housing sleeve (2) for friction-fitted fixing of the storage means (3) in the housing sleeve (2),
**characterized in that**
in order to close the housing sleeve (2), a plug sleeve (17) is provided which can be inserted into the rear sleeve region (10) and **in that** fixing projections which are provided with terminal feed-in stops (21) and are formed as ribs (20) are provided in the plug sleeve (17) for terminal fixing of the storage means (3) .

2. The inhalation stick as claimed in claim 1, **characterized in that** a plurality of fixing projections are provided in the housing sleeve (2) and/or **in that** the storage means (3) bears exclusively against the fixing projections without bearing against the remaining inner wall regions (8) of the compartment (4) and/or **in that** the storage means (3) is disposed centrally in the compartment (4) by fixing projections which are distributed uniformly about the circumference and are preferably formed as elongate ribs (7).

3. The inhalation stick as claimed in claim 1 or claim 2, **characterized in that** the housing sleeve (2) is provided with a front sleeve region (9) in particular formed as an inhalation region and a rear sleeve region (10) in particular formed as a handle region and/or **in that** at least one, in particular central, nose opening (12) which leads into the compartment (4) is provided on the front end (11) of the front sleeve region (9), and **in that** at least one lateral opening (13) which leads into the compartment (4), in particular radially, is provided, preferably on the front sleeve region (9).

4. The inhalation stick as claimed in one of the preceding claims, **characterized in that** the ribs (7) are provided in the region of the front end (11) of the front sleeve region (9) and **in that** preferably, the ribs (7) each have a stop (14) for terminal seating and fixing of the storage means (3) in the axial direction and a terminal feed-in bevel (16).

5. The inhalation stick as claimed in one of the preceding claims, **characterized in that** the plug sleeve (17) is snap fitted with the housing sleeve (2) and wherein preferably, a snap fitting projection (22), in particular circumferential, is provided externally on the plug sleeve (17) and a circumferential snap fitting step (23) is provided on the interior of the rear sleeve region (10), and/or **in that** a closure cap (24) is provided for positioning on the front sleeve region (9) in order to close the nose and lateral openings (12, 13) and **in that** preferably, a screw connection is provided at the transition from the front sleeve region (9) to the rear sleeve region (10) in order to screw the closure cap (24) together with the housing sleeve (2).

6. The inhalation stick as claimed in one of the preceding claims, **characterized in that** the inhalation stick (1) contains a composition for use in the prophylactic and/or therapeutic treatment of diseases, wherein the interior of the inhalation stick comprises a storage means, preferably a textile storage means, wherein the storage means is provided and/or charged with the composition, and
- wherein the composition comprises at least two essential oils which differ from each other, in particular at least three essential oils which each differ from each other, wherein the essential oils are selected from the group formed by:
bergamot oil (Citrus aurantium var. bergamia),
cedarwood oil (Cupressus funebris Endlicher),
cypress oil (Cupressus sempervirens),
eucalyptus oil (Eucalyptus globulus),
geranium oil (Pelargonium graveolens),
gingergrass oil (Cymbopogon martinii var. sofia),
lavender oil (Lavandula angustifolia),
marjoram oil (Origanum majorana),
mandarin oil (Citrus reticulata),
clary sage oil (Salvia sclarea),
orange oil (Citrus aurantium var. dulcis),
peppermint oil (Mentha piperita),
pepper oil (Piper nigrum),
rosemary oil (Rosmarinus officinalis),
thyme oil (Thymus serpyllum),
vetiver oil (Vetiveria zizanoides),
juniper berry oil (Juniperus communis L.),
cinnamon bark oil (Cinnamomum ceylanicum),
lemon oil (Citrus limon),
grapefruit oil (Citrus decumana),
basil oil (Ocimum basilicum),
spruce oil (Picea excelsa)
as well as mixtures and combinations thereof.

7. The inhalation stick as claimed in claim 6,
wherein the storage means is porous and air-permeable in configuration; and/or wherein the storage means comprises a textile material and/or is constituted thereby, in particular based on cotton, cellulose or cellulose derivatives, preferably cellulose acetates, glycerin triacetates as well as mixtures and combinations thereof; and/or
wherein the storage means has a water absorbency of at least 3 g/g, in particular at least 4 g/g, preferably at least 5 g/g; and/or
wherein the storage means is shaped as a cylinder or as a rod, in particular with lengths of 10 to 100 mm and/or with thicknesses (diameters) of 1 to 20 mm; and/or
wherein the storage means has a weight of 0.1 to 10.0 g, in particular 0.2 to 7.5 g, preferably 0.3 to 5 g.

8. The inhalation stick as claimed in claim 6 or claim 7, wherein the inhalation stick and/or the storage means contains or contain the composition in effective quantities, in particular therapeutically effective quantities; and/or
wherein the inhalation stick and/or the storage means contains or contain the composition in quantities of 0.01 to 10.0 mL, in particular 0.1 to 5 mL, preferably 0.2 to 1 mL; and/or
wherein the inhalation stick and/or the storage means contains or contain the composition in quantities of 0.005 to 15 g, in particular 0.05 to 7.5 g, preferably 0.01 to 1.5 g, particularly preferably 0.1 to 1 g.

9. The inhalation stick as claimed in one of claims 6 to 8,
wherein the inhalation stick and/or the storage means contains or contain the composition in relative quantities of 0.1% to 300% by weight, in particular 0.2% to 150% by weight, preferably 0.5% to 100% by weight, particularly preferably 1% to 80% by weight with respect to the storage means, and/or
wherein upon application by inhalation, the storage means is capable of releasing the composition in effective, in particular therapeutically effective quantities.

10. The inhalation stick as claimed in one of claims 6 to 9,
wherein the composition has a density (relative density), at 20°C and at atmospheric pressure (101.325 Pascal), in the range 0.5 to 1.5 g/cm³, in particular in the range 0.6 to 1.2 g/cm³, preferably in the range 0.7 to 1.1 g/cm³; and/or
wherein the composition is volatile and/or is present in the liquid physical state at 20°C and at atmospheric pressure (101.325 Pascal); and/or
wherein at 20°C, the composition has a refractive index in the range 1300 to 1700, in particular in the range 1350 to 1650, preferably in the range 1400 to 1600.

11. The inhalation stick as claimed in one of claims 6 to 10,
wherein the composition comprises a proportion of VOC (volatile organic compounds) of less than 1% by weight, in particular of less than 0.5% by weight, preferably 0% by weight, with respect to the composition; and/or
wherein the composition is obtained by compression and/or steam distillation, in particular from the corresponding starting drugs and/or plant parts such as fruit peel, fruit, wood, leaves, branches, twig tips, flowers, inflorescences, grass, herbs, roots, rind, or the like; and/or
wherein the composition is volatile and/or fugitive, in particular wherein the composition has a vapour pressure at 20°C of more than 20 mbar, in particular more than 24 mbar, preferably more than 28 mbar, preferably more than 30 mbar, particularly preferably more than 40 mbar, more particularly preferably more than 60 mbar, yet more preferably more than 100 mbar.

12. The inhalation stick as claimed in one of claims 6 to 11,
wherein the composition comprises at least one of the following combinations of essential oils:
(i) peppermint oil/rosemary oil/lavender oil, in particular in weight ratios of 40-80/10-50/1-20, preferably for the prophylactic and/or therapeutic treatment of chronic pain;
(ii) grapefruit oil/peppermint oil, in particular in weight ratios of 30-70/30-70, preferably for the prophylactic and/or therapeutic treatment of metabolic diseases, in particular diabetes or obesity;
(iii) eucalyptus oil/peppermint oil/thyme oil, in particular in weight ratios of 60-90/5-20/5-20, preferably for the prophylactic and/or therapeutic treatment of infections, in particular colds, rhinitis and bronchitis;
(iv) lavender oil/vetiver oil/mandarin oil, in particular in weight ratios of 20-50/20-50/15-40, preferably for the prophylactic and/or therapeutic treatment of cardiovascular diseases (diseases of the heart/circulatory system), in particular high blood pressure (hypertonia), and/or psychosomatic diseases, in particular stress, physical-mental strain, withdrawal syndromes and insomnia;
(v) lemon oil/peppermint oil/rosemary oil/grapefruit oil/pepper oil/basil oil, in particular in weight ratios of 15-30/15-30/15-30/10-25/2-10/1-8;
(vi) bergamot oil/lemon oil/cedarwood oil/clary sage oil/vetiver oil, in particular in weight ratios of 25-40/20-35/10-20/10-20/5-20, preferably for the prophylactic and/or therapeutic treatment of cardiovascular diseases (diseases of the heart/circulatory system), in particular high blood pressure (hypertonia), and/or psychosomatic diseases, in particular stress, physical-mental strain, withdrawal syndromes and insomnia;
(vii)peppermint oil/vetiver oil/juniper berry oil/thyme oil, in particular in weight ratios of 25-45/20-40/20-40/1-15;
(viii) peppermint oil/cypress oil/geranium oil/gingergrass oil, in particular in weight ratios of 40-60/25-45/10-30/1-10;
(ix) peppermint oil/rosemary oil/cinnamon bark oil, in particular in weight ratios of 65-95/1-15/1-10, preferably for the prophylactic and/or therapeutic treatment of cardiovascular diseases (diseases of the heart/circulatory system), in particular high blood pressure (hypertonia), and/or psychosomatic diseases, in particular stress, physical-mental strain, withdrawal syndromes and insomnia;
(x) peppermint oil/grapefruit oil/rosemary oil, in particular in weight ratios of 75-95/1-12/1-5;
(xi) bergamot oil/marjoram oil/lavender oil/orange oil, in particular in weight ratios of 30-50/10-30/10-30/10-30;
(xii)rosemary oil/peppermint oil/basil oil, in particular in weight ratios of 40-60/35-55/1-15.

13. A method for assembling an inhalation stick (1) as defined in one of the preceding claims, wherein the storage means (3) in the uncharged state is initially inserted into the compartment (4), wherein the compartment (4) is subsequently closed and wherein after closing the compartment, the liquid is then introduced into the storage means (3) via the nose opening (12) and/or the lateral opening (13).

14. The method according to claim 13, wherein a partial vacuum is applied and/or provided, in particular at least in the housing sleeve (2), at least during introduction of the liquid into the storage means (3).

15. The method according to claim 13 or claim 14, wherein the storage means (3) is initially inserted into the compartment (4) in the uncharged state, wherein the compartment (4) is subsequently closed by inserting the plug sleeve (17) into the rear sleeve region (10) and wherein the storage means (3) is fixed at both ends in the longitudinal direction in its final installation position by means of the inserted plug sleeve (17).

## Revendications

1. Dispositif d'inhalation (1) destiné à l'inhalation par le nez comprenant un manchon de logement (2) étiré en longueur et un moyen de stockage (3) en forme de tube pour stocker au moins une huile essentielle, dans lequel le manchon de logement (2) présente un espace de réception intérieur (4) pour recevoir le moyen de stockage (3),
dans lequel à l'intérieur du manchon de logement (2), au moins une saillie de fixation faisant saillie dans le sens radial dans l'espace de réception (4) est prévue pour la fixation par friction du moyen de stockage (3) dans le manchon de logement (2),
**caractérisé en ce que**
pour fermer le manchon de logement (2), un manchon-bouchon (17) pouvant être inséré dans la partie arrière du manchon (10) est prévu, et que des saillies de fixation avec des butées à plan incliné latérales (21) à l'extrémité, formées en tant que nervures (20) sont prévues dans le manchon-bouchon (17) pour la fixation du moyen de stockage (3) à l'extrémité.

2. Dispositif d'inhalation (1) selon la revendication 1, **caractérisé en ce qu'**une pluralité de saillies de fixation est prévue dans le manchon de logement (2) et/ou que le moyen de stockage (3) repose exclusivement sur les saillies de fixation, sans reposer sur les parties de paroi intérieure restantes (8) de l'espace de réception (4) et/ou que le moyen de stockage (3) est disposé centré dans l'espace de réception (4) par en particulier des saillies de fixation formées de préférence en tant que nervures (7) étirées en longueur et réparties uniformément sur le pourtour.

3. Dispositif d'inhalation (1) selon la revendication 1 ou 2, **caractérisé en ce que** le manchon de logement (2) présente une partie de manchon avant (9) formée en particulier en tant que zone d'inhalation et une partie de manchon arrière (10) formée en particulier en tant que zone de préhension et/ou que sur l'extrémité avant (11) de la partie de manchon avant (9), au moins une ouverture nasale (12) en particulier centrale conduisant dans l'espace de réception (4) est prévue, et que de préférence, sur la partie de manchon avant (9), au moins une ouverture latérale (13) conduisant radialement dans l'espace de réception (4) est prévue.

4. Dispositif d'inhalation selon l'une des revendications précédentes, **caractérisé en ce que** les nervures (7) sont prévues au niveau de l'extrémité avant (11) de la partie de manchon avant (9) et que de préférence, les nervures (7) présentent respectivement une butée (14) pour un appui et une fixation d'extrémité du moyen de stockage (3) dans le sens axial et un biseau (16) d'extrémité.

5. Dispositif d'inhalation selon l'une des revendications précédentes, **caractérisé en ce que** le manchon-bouchon (17) est encliqueté avec le manchon de logement (2) et que de préférence, une saillie d'encliquetage (22) en particulier périphérique est prévue sur l'extérieur du manchon-bouchon (17) et qu'un cran d'encliquetage (23) périphérique est prévu sur l'intérieur de la partie de manchon arrière (10) et/ou
qu'un bouchon de fermeture (24) destiné à être placé sur la partie de manchon avant (9) pour fermer les ouvertures nasale et latérale (12, 13) est prévu et que de préférence, une liaison vissée pour visser le bouchon de fermeture (24) au manchon de logement (2) est prévue sur le passage de la partie de manchon avant (9) à la partie de manchon arrière (10).

6. Dispositif d'inhalation selon l'une des revendications précédentes, **caractérisé en ce que** le dispositif d'inhalation (1) contient une composition destinée à être utilisée pour le traitement prophylactique et/ou thérapeutique de maladies, dans lequel le dispositif d'inhalation présente à l'intérieur un moyen de stockage, de préférence un moyen de stockage textile, dans lequel le moyen de stockage est doté de et/ou est alimenté avec la composition et
- dans lequel la composition présente au moins deux huiles essentielles différentes, en particulier au moins trois huiles essentielles différentes, dans lequel les huiles essentielles sont sélectionnées parmi le groupe suivant
huile essentielle de bergamote (Citrus aurantium var. bergamia),
huile essentielle de bois de cèdre (Cupressus funebris Endlicher),
huile essentielle de cyprès (Cupressus sempervirens),
huile essentielle d'eucalyptus (Eucalyptus graveolens),
huile essentielle de géranium (Pelargonium graveolens),
huile essentielle de gingergrass (Cymbopogon martinii var. sofia),
huile essentielle de lavande (Lavandula angustifolia),
huile essentielle de marjolaine (Origanum majorana),
huile essentielle de mandarinier (Citrus reticulata),
huile essentielle de sauge sclarée (Salvia sclarea),
huile essentielle d'orange douce (Citrus aurantium var. dulcis)
huile essentielle de menthe poivrée (Mentha piperita),
huile essentielle de poivre noir (Piper nigrum),
huile essentielle de romarin (Rosmarinus officinalis),
huile essentielle de thym serpolet (Thymus serpyllum),
huile essentielle de vétiver (Vetiveria zizanoides),
huile essentielle de genévrier (Juniperus communis L.),
huile essentielle d'écorce de cannelier de Ceylan (Cinnamomum ceylanicum),
huile essentielle de citron (Citrus limon),
huile essentielle de pamplemousse (Citrus decumana),
huile essentielle de basilic (Ocimum basilicum),
huile essentielle d'épicéa (Picea excelsa)
ainsi que leurs mélanges et combinaisons.

7. Dispositif d'inhalation selon la revendication 6, dans lequel le moyen de stockage est poreux et/ou perméable à l'air ; et/ou
dans lequel le moyen de stockage comprend un matériau textile et/ou est fabriqué dedans, en particulier à base de coton, de cellulose ou de dérivés de cellulose, de préférence des acétates de cellulose, des triacétates de glycérine ainsi que leurs mélanges et combinaisons ; et/ou
dans lequel le moyen de stockage présente un pouvoir d'absorption d'eau d'au moins 3 g/g, en particulier d'au moins 4 g/g, de préférence d'au moins 5 g/g ; et/ou
dans lequel le moyen de stockage est cylindrique ou tubulaire, en particulier avec des longueurs de 10 à 100 mm et/ou des épaisseurs (diamètres) de 1 à 20 mm ; et/ou
dans lequel le moyen de stockage présente un poids de 0,1 à 10 g, en particulier de 0,2 à 7,5 g, de préférence de 0,3 à 5 g.

8. Dispositif d'inhalation selon la revendication 6 ou 7,
dans lequel le dispositif d'inhalation et/ou le moyen de stockage contient/contiennent la composition dans des quantités actives, en particulier actives sur le plan thérapeutique ; et/ou
dans lequel le dispositif d'inhalation et/ou le moyen de stockage contient/contiennent la composition dans des quantités de 0,01 à 10 ml, en particulier de 0,1 à 5 ml, de préférence de 0,2 à 1 ml ; et/ou
dans lequel le dispositif d'inhalation et/ou le moyen de stockage contient/contiennent la composition dans des quantités de 0,005 à 15 g, en particulier de 0,05 à 7,5 g, de préférence de 0,01 à 1,5 g, particulièrement de préférence de 0,1 à 1g.

9. Dispositif d'inhalation selon l'une des revendications 6 à 8,
dans lequel le dispositif d'inhalation et/ou le moyen de stockage contient/contiennent la composition dans des quantités relatives de 0,1 à 300 % en poids, en particulier de 0,2 à 150 % en poids, de préférence de 0,5 à 100 % en poids, particulièrement de préférence de 1 à 80 % en poids, rapporté au moyen de stockage ; et/ou
dans lequel le moyen de stockage est en mesure de libérer la composition dans des quantités actives, en particulier actives sur le plan thérapeutique lors d'applications d'inhalation.

10. Dispositif d'inhalation selon l'une des revendications 6 à 9,
dans lequel la composition présente à 20°C et une pression atmosphérique (101.325 Pascal), une densité (densité relative) dans la plage de 0,5 à 1,5 g/cm³, en particulier dans la plage de 0,6 à 1,2 g/cm³, de préférence dans la plage de 0,7 à 1,1 g/cm³ ; et/ou dans lequel la composition, à 20°C et une pression atmosphérique (101.325 Pascal), est fluidique et/ou est présente en état d'agrégat fluidique ; et/ou dans lequel la composition présente à 20°C un indice de réfraction dans la plage de 1,300 à 1,700, en particulier dans la plage de 1,350 à 1,650, de préférence dans la plage de 1,400 à 1,600.

11. Dispositif d'inhalation selon l'une des revendications 6 à 10,
dans lequel la composition présente une teneur en COV (Composés Organiques Volatils) inférieure à 1 % en poids, en particulier inférieure à 0,5 % en poids, de préférence de 0 % en poids rapporté à la composition ; et/ou
dans lequel la composition est obtenue par pressage et/ou distillation à la vapeur d'eau, en particulier à partir des drogues de départ et/ou parties de plantes correspondantes comme des écorces de fruits, des fruits, du bois, des feuilles, des branches, des pointes de branches, des fleurs, des inflorescences, de l'herbe, des herbes, des racines, des écorces ou similaires ; et/ou
dans lequel la composition est volatile, en particulier dans lequel la composition présente à 20°C une pression de vapeur supérieure à 20 mbar, en particulier supérieure à 24 mbar, de préférence supérieure à 28 mbar, de préférence supérieure à 30 mbar, particulièrement de préférence supérieure à 40 mbar, tout particulièrement de préférence supérieure à 60 mbar, plus encore de préférence supérieure à 100 mbar.

12. Dispositif d'inhalation selon l'une des revendications 6 à 11,
dans lequel la composition présente au moins une des combinaisons suivantes d'huiles essentielles :
(i) huile essentielle de menthe poivrée/ huile essentielle de romarin/ huile essentielle de lavande, en particulier dans des rapports liés au poids de 40-80/10-50/1-20, de préférence pour le traitement prophylactique et/ou thérapeutique d'états douloureux ;
(ii) huile essentielle de pamplemousse/ huile essentielle de menthe poivrée, en particulier dans des rapports liés au poids de 30-70/30-70, de préférence pour le traitement prophylactique et/ou thérapeutique de maladies du métabolisme, en particulier le diabète ou le surpoids ;
(iii) huile essentielle d'eucalyptus/huile essentielle de menthe poivrée/huile essentielle de thym, en particulier dans des rapports liés au poids de 60-90/5-20/5-20, de préférence pour le traitement prophylactique et/ou thérapeutique d'infections, en particulier de rhumes, de rhinite et de bronchite ;
(iv) huile essentielle de lavande/huile essentielle de vétiver/huile essentielle de mandarinier, en particulier dans des rapports liés au poids de 20-50/20-50/15-40, de préférence pour le traitement prophylactique et/ou thérapeutique de maladies cardio-vasculaires (maladies du coeur/de la circulation sanguine), en particulier hypertension (hypertonie), et/ou maladies psychosomatiques, en particulier le stress, les surcharges physico-psychiques, les syndromes du sevrage et les troubles du sommeil ;
(v) huile essentielle de citron/huile essentielle de menthe poivrée/huile essentielle de romarin/huile essentielle de pamplemousse/huile essentielle de poivre noir/huile essentielle de basilic, en particulier dans des rapports liés au poids de 15-30/15-30/15-30/10-25/2-10/1-8 ;
(vi) huile essentielle de bergamote/huile essentielle de citron/huile essentielle de bois de cèdre/huile essentielle de sauge sclarée/huile essentielle de vétiver, en particulier dans des rapports liés au poids de 25-40/20-35/10-20/10-20/5-20, de préférence pour le traitement prophylactique et/ou thérapeutique de maladies cardio-vasculaires (maladies du coeur/de la circulation sanguine), en particulier hypertension (hypertonie), et/ou maladies psychosomatiques, en particulier le stress, les surcharges physico-psychiques, les syndromes du sevrage et les troubles du sommeil ;
(vii) huile essentielle de menthe poivrée/ huile essentielle de vétiver/ huile essentielle de genévrier/ huile essentielle de thym, en particulier dans des rapports liés au poids de 25-45/20-40/20-40/1-15 ;
(viii) huile essentielle de menthe poivrée/ huile essentielle de cyprès/ huile essentielle de géranium/ huile essentielle de gingergrass, en particulier dans des rapports liés au poids de 40-60/25-45/10-30/1-10 ;
(ix) huile essentielle de menthe poivrée/ huile essentielle de romarin/ huile essentielle d'écorce de cannelier de Ceylan, en particulier dans des rapports liés au poids de 65-95/1-15/1-10, de préférence pour le traitement prophylactique et/ou thérapeutique de maladies cardio-vasculaires (maladies du coeur/de la circulation sanguine), en particulier hypertension (hypertonie), et/ou maladies psychosomatiques, en particulier le stress, les surcharges physico-psychiques, les syndromes du sevrage et les troubles du sommeil ;
(x) huile essentielle de menthe poivrée/ huile essentielle de pamplemousse/huile essentielle de romarin, en particulier dans des rapports liés au poids de 75-95/1-12/1-5 ;
(xi) huile essentielle de bergamote/ huile essentielle de marjolaine/ huile essentielle de lavande/ huile essentielle d'orange, en particulier dans des rapports liés au poids de 30-50/10-30/10-30/10-30 ;
(xii) huile essentielle de romarin/huile essentielle de menthe poivrée/huile essentielle de basilic, en particulier dans des rapports liés au poids de 40-60/35-55/1-15.

13. Procédé de montage d'un dispositif d'inhalation (1) défini comme dans l'une des revendications précédentes, dans lequel le moyen de stockage (3) dans l'état non chargé est d'abord placé dans l'espace de réception (4), dans lequel l'espace de réception (4) est ensuite fermé et dans lequel le fluide est introduit dans le moyen de stockage (3) par l'ouverture nasale (12) et/ou l'ouverture latérale (13) après la fermeture de l'espace de réception.

14. Procédé selon la revendication 13, dans lequel au moins pendant l'introduction du fluide dans le moyen de stockage (3), une dépression, en particulier au moins dans le manchon de logement (2) est créée et/ou est prévue.

15. Procédé selon la revendication 13 ou 14, dans lequel le moyen de stockage (3) dans l'état non chargé est d'abord placé dans l'espace de réception (4), dans lequel l'espace de réception (4) est ensuite fermé par introduction du manchon-bouchon (17) dans la partie de manchon arrière (10) et dans lequel le moyen de stockage (3) dans son montage final est fixé aux deux extrémités dans le sens longitudinal du fait que le manchon-bouchon (17) est rentré.
